# EUROPEAN PATENT SPECIFICATION

(11) **EP 0 736 027 B1**
(45) Date of publication and mention of the grant of the patent: **29.05.2002**
(21) Application number: 95906679.6
(22) Date of filing: 22.12.1994
(51) Int. Cl.: C07D 473/40, C07D 473/18

(54) **GUANINE SYNTHONS FOR PEPTIDE NUCLEIC ACID SYNTHESIS AND METHOD FOR PRODUCTION**
GUANINSYNTHONE FÜR DIE SYNTHESE VON NUCLEINSÄUREN UND VERFAHREN ZU DEREN HERSTELLUNG
SYNTHONS DE GUANINE UTILISES DANS LA SYNTHESE DES ACIDES NUCLEIQUES PEPTIDIQUES ET LEUR PROCEDE DE FABRICATION

(30) Priority: 22.12.1993 US 172695
(43) Date of publication of application: 09.10.1996
(73) Proprietor: PERSEPTIVE BIOSYSTEMS, INC., Framingham, MA 07101 (US)
(72) Inventor: COULL, James, M., Westford, MA 01886 (US); HODGE, Richard, P., Dracut, MA 01826 (US)
(74) Representative: Kirkham, Nicholas Andrew
(86) International application number: US9414742
(87) International publication number: WO9517403

(56) References cited:
- WO-A-92/20702
- WO-A-92/20703
- WO-A-93/12129
- JOURNAL OF HETEROCYCLIC CHEMISTRY, vol.12, no.2, April 1975, UTAH pages 273 - 278 J. VIDAL-GOMEZ ET AL
- JOURNAL OF SYNTHETIC ORGANIC CHEMISTRY, no.1, January 1991, NEW YORK pages 157 - 159 PAUL R. BIRKETT ET AL
- JOURNAL OF THE AMERICAN CHEMICAL SOCIETY, vol.104, no.21, 20 October 1982 pages 5702 - 5708 BRUCE E. WATKINS ET AL cited in the application
- THE JOURNAL OF ORGANIC CHEMISTRY, vol.47, no.23, 5 November 1982 pages 4471 - 4477 BRUCE E. WATKINS ET AL cited in the application

## Description

### Field of the Invention

This invention relates to methods for preparing novel guanine synthons for Peptide Nucleic Acid (PNA) synthesis.

### Description of the Background Art

Peptide Nucleic Acids (PNAs) are synthetic polyamides which are promising candidates for the sequence-specific regulation of DNA expression and for the preparation of gene targeted drugs. See WO-A-9 220 702 and WO-A-9 220 703. PNAs are Biopolymer hybrids which possess a peptide-like backbone to which the nucleobases of DNA are attached. Specifically, PNAs are synthetic polyamides comprised of repeating units of the amino add, N-(2-aminoethyl)-glycine, to which the nucleobases adenine, cytosine, guanine and thymine are attached through a methylene carbonyl group.

PNAs are currently synthesized from monomers protected according to the t-boc/benzyl protection strategy wherein the backbone amino group of the growing polymer is protected with the t-butyloxycarbonyl (t-boc) group and the exocyclic amino groups of the nucleobases are protected with the benzyloxycarbonyl benzyl) group.
See WO-A-9 220 702 and WO-A-9 220 703. Certain reported guanine PNA synthons are protected as benzyl ethers at the C6 carbonyl group but optionally possess benzyl protection of the exocyclic 2-amino group. Compare WO-A-9 220 702 and WO-A-9 312 129.
Given the relative reactivity of the C6 carbonyl group (enol form) and the more reactive exocyclic 2-amino group, there exists no compelling reason for protecting the C6 carbonyl group during PNA synthesis whereas protection of the more reactive 2-amino group is preferred.

The benzyloxycarbonyl group has been utilized in DNA synthesis for the protection of the exocyclic amino groups of the nucleobases cytosine, adenine and guanine. See Watkins et al, J. Org. Chem., (1982) 47:4471-77 and Watkins et al., J. Am. Chem. Soc., (1982) 104:5702-08. Nonetheless, the guanine synthon was difficult to prepare because the exocyclic 2-amino group of guanine was not reactive toward reagents routinely used to introduce the benzyl group. Id. These include, benzyl chloroformate, benzyloxycarbonyl imidazole and acyl imidazolium salts of benzyloxycarbonyl imidazole. Consequently, a non-conventional multi-step procedure was described wherein treatment with phenyl chlorothioformate simultaneously protected both the C6 carbonyl group and the exocyclic 2-amino group. Thereafter, the adduct was converted to a carbamate protected guanine compound whereby the C6 carbonyl protecting group was subsequently removed. Nonetheless, this indirect method is laborious because it requires the formation of a carbamate protecting group from the initial adduct and the subsequent deprotection of the C6 carbonyl group.

Suitably protected derivatives of 2-amino-6-chloropurine may be converted to guanine compounds by displacement of the 6-chloro group with oxygen nucleophiles. See Robins et al, J. Am. Chem. Soc. (1965) 87:4934, Reese et al., Nucl. Adds Res., (1981) 9: 4611 and Hodge et al., J. Org. Chem., (1990) 56:1553. Indeed, suitably protected derivatives of 2-amino-6-chloropurine are among the starting materials currently described for preparation of the reported guanine PNA synthons. See WO-A-9 220 702 and WO-A-9 312 129.

The inventors of PNA describe a guanine synthon having no protection of the exocyclic 2-amino group but having the C6 carbonyl group protected as a benzyl ether. See W-A-9 220 702. This protection strategy is surprising because the more reactive 2-amino group will likely react (at least marginally) with the activated carboxylic acid group of other PNA monomers, thereby causing branching of the synthesized polymer. Conversely, the enol, which exists when the C6 carbonyl group remains unprotected, is not reactive enough to result in polymer branching and therefore should require no protection. This particular approach is inconsistent with t-boc/benzyl protection strategy they employed for the other PNA synthons.

In a more recent patent application, the guanine PNA synthon has both benzyl protection of the exocyclic 2-amino group and a C6 carbonyl group protected as a benzyl ether. See WO-A-9 312 129. As previously discussed, there is no compelling rationale for protecting the C6 carbonyl group of the guanine PNA synthon. However, protection of the C6 carbonyl group enables selective ionization of the exocyclic 2-amino group of the guanine heterocycle thereby facilitating the reaction of the ionized 2-amino group with conventional benzyl protecting reagents (e.g. benzyloxycarbonyl imidazole). Nonetheless, protection of the exocyclic 2-amino group occurs on a guanine derivative additionally protected at the C6 carbonyl group of the nucleobase. Thus, the resulting synthon has both exocyclic 2-amino group and C6 carbonyl group protection. Hence, there remains no reported convenient high yield synthesis of a guanine PNA synthon having selective benzyl protection of the exocyclic 2-amino group, wherein the C6 carbonyl group remains unprotected.

J. Am. Chem. Soc. 1982, 104, 5702-5708 discloses synthesis of oligodeoxyribonucleotides using *N*-benzyloxycarbonyl-blocked nucleosides. The exo amino groups of 2'-deoxyadenosine and 2'-deoxycytidine have been blocked as the benzyl carbamates, and 2'-deoxyguanosine has been blocked as its 2-*N*-(benzyloxycarbonyl)carbamate and 6-*O*-benzyl ether. These blocked nucleosides have been incorporated into an efficient oligodeoxyribonucleotide synthetic scheme, and the resulting oligomer has been successfully deblocked by using transfer hydrogenation. The deblocking conditions result in no reduction of the pyrimidine bases.

J. Org. Chem. 1982, 47, 4471-4477 discloses the synthesis of benzyl and benzyloxycarbonyl base-blocked 2'-deoxyribonucleosides. Acylimidazoles have been alkylated with trialkyloxonium tetrafluoroborates to form acylimidazolium salts. These salts, particularly (benzyloxycarbonyl)imidazolium salts, are shown to be effective agents for the direct, mono-N-protection of deoxynucleosides as their acyl derivatives. These acyl nucleosides are also available via thiocarbamate intermediates. Thus 3',5'-bis(tert-butyldimethylsilyl)-2'-deoxyguanosine (4b), on treatment with phenyl chlorothioformate, gave the 6-thiophenyl-substituted purine 38a. The thiophenyl group of 38a can be replaced by hydrogen, amino, and alkoxy groups to give a variety of substituted purine deoxyribonucleosides.

WO-A-9312129 discloses peptide nucleic acids and their effect on genetic material. Peptide nucleic acids oligomers of a particular formula are disclosed and their use in affecting genetic material, e.g. as triplex or antisense in the treatment of disease.

WO-A-9220702 discloses that a class of compounds, known as peptide nucleic acids, bind complementary ssDNA and RNA strands more strongly than a corresponding DNA. The peptide nucleic acids generally comprise ligands such as naturally occurring DNA bases attached to a peptide backbone through a suitable linker.

WO-A-9220703 discloses the use of nucleic acid analogues in diagnostics and analytical procedures. Methods of capture, recognition, detection, identification or quantitation of nucleic acids and diagnostics uses generally are described in which are used; (a) a peptide nucleic acid (PNA) comprising a polyamide backbone bearing a plurality of ligands at respective spaced locations along said backbone, said ligands being each independently naturally occurring nucleobases, non-naturally occurring nucleobases or nucleobase-binding groups, each said ligand being bound directly or indirectly to a nitrogen atom in said backbone, and said ligand bearing nitrogen atoms mainly being separated from one another in said backbone by from 4 to 8 intervening atoms; (b) a nucleic acid analogue capable of hybridising to a nucleic acid of complementary sequence to form a hybrid which is more stable against denaturation by heat than a hybrid between the conventional deoxyribonucleotide corresponding to said analogue and said nucleic acid; or (c) a nucleic acid analogue capable of hybridising to a double stranded nucleic acid in which one strand has a sequence complementary to said analogue, so as to displace the other strand from said one strand. Preferred compounds are also disclosed.

### Summary of the Invention

This invention is a method for a convenient high yield synthesis of novel guanine PNA synthons having selective carbamate protection of the exocyclic 2-amino group. Generally, the guanine PNA synthon is assembled by coupling the selectively carbamate protected guanine side chain moiety to a suitably N-protected backbone ester of the amino acid, N-(2-aminoethyl)-glycine, followed by hydrolysis of the backbone ester group. Because only the exocyclic 2-amino group of the nucleobase is benzyl protected, the preferred guanine monomer of this invention is selectively protected and therefore completely consistent with the t-boc/benzyl protection strategy currently employed for PNA synthesis.

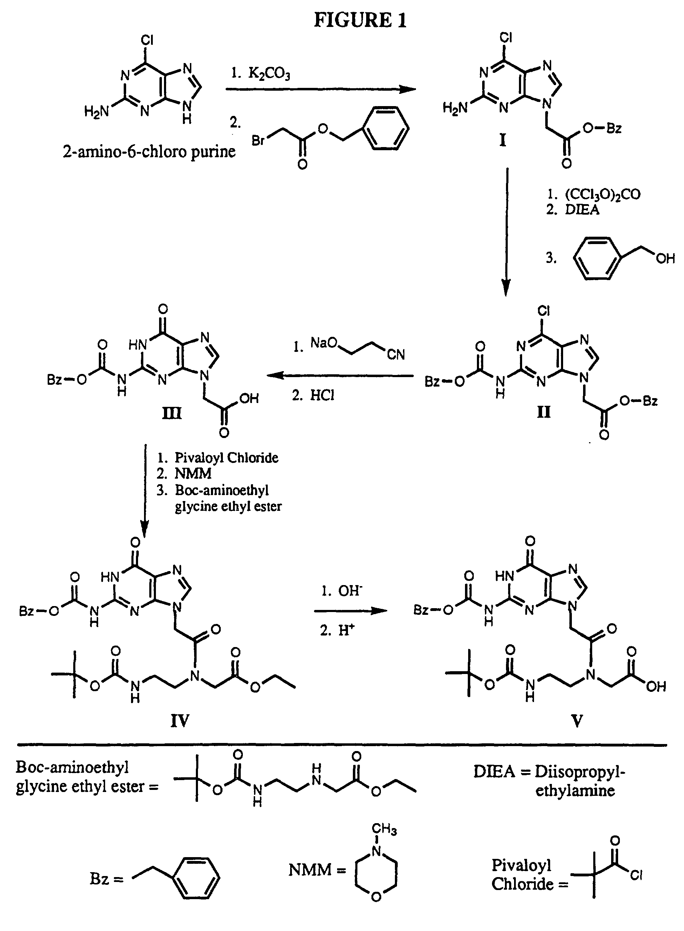

By the method of this invention, partially protected 2-amino-6-halopurine compounds (e.g. compound I, Figure 1), which are alkylated at the N9 nitrogen atom with acetate ester moieties, are reacted with phosgene in the presence of a non-nucleophilic base. This step converts the relatively non-nucleophilic exocyclic 2-amino group of the purine to a reactive electrophilic 2-isocyanate group. The intermediary 2-isocyanato-6-halopurine is not isolated but is reacted with an alcohol thereby forming a fully protected 2-amino-6-chloropurine having a carbamate protected exocyclic 2-amino group (e.g. compound II). Thus, applicants demonstrate a convenient high yield synthesis of a selectively protected compound which can be directly converted to a selectively carbamate protected guanine side chain moiety suitable for coupling to a suitably N-protected backbone ester of the amino acid N-(2-aminoethyl)-glycine.

Generally, the fully protected 2-amino-6-halo-purine is converted to a carbamate protected guanine side chain moiety by simultaneously converting the 6-halo group to a C6 carbonyl group and hydrolyzing the N9 acetate ester group. Preferably, both transformations are accomplished in a single reaction by treatment with the alkoxide of 3-hydroxypropionitrile. Thus, the fully protected 2-amino-6-halopurine is treated with at least three equivalents of the alkoxide of 3-hydroxypropionitrile. Two equivalents of alkoxide are consumed to convert the 6-halo group to a C6 carbonyl group and at least one equivalent is consumed to hydrolyze the N9 acetate ester group thereby forming the desired selectively carbamate protected guanine side chain moiety (e.g. compound III).

Thus, the components of the guanine PNA synthon are assembled by coupling the carbamate protected guanine side chain moiety to the suitably N-protected backbone ester. Preferably, the suitably N-protected backbone ester is N¹-(*tert*-butyloxycarbonyl)-N⁴-(2-aminoethyl)-glycine ethyl ester which is coupled to the carbamate protected guanine side chain moiety, thereby forming the guanine PNA synthon ester (e.g. compound IV). The synthesized guanine PNA synthon ester is then converted to a free carboxylic acid by hydrolyzing the backbone ester group. Consequently, the method yields a novel guanine PNA synthon having selective carbamate protection of the exocyclic 2-amino group wherein the C6 carbonyl group remains unprotected. With reference to Figure 1, the guanine PNA synthon is compound V.

The invention is also directed to a fully protected 2-amino-6-halopurine compound of the general formula: 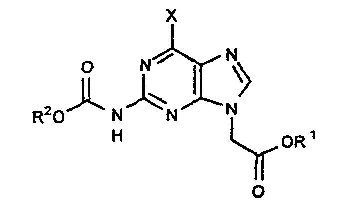 wherein
X is a halogen atom and is selected from the group consisting of fluorine (F), chlorine (Cl), bromine (Br) and iodine (I); R¹ is selected from the group consisting of methyl, ethyl, 2,2,2-trichloroethyl, 2-(trimethylsilyl)-ethyl, 2-(phenylthio)-ethyl, propyl, isopropyl, n-butyl, t-butyl, allyl, 1-isopropyl allyl, cinnamyl, 4-nitrocinnamyl and a benzyl group or derivative thereof of the formula: 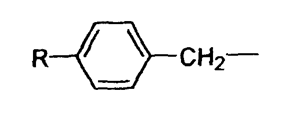 wherein R is hydrogen, isopropyl, t-butyl, or methoxy. R² is independently selected from the group consisting of methyl, ethyl, 2,2,2-trichloroethyl, 2-(trimethylsilyl)-ethyl, propyl, isopropyl, n-butyl, t-butyl, allyl, 1-isopropyl allyl, cinnamyl, 4-nitrocinnamyl, diphenylmethyl, or a benzyl group or derivative thereof (as defined above).

In another embodiment, R² can be a group having the formula:

R⁵SCH₂CH₂-

wherein R⁵ is selected from the group consisting of methyl, ethyl, propyl, isopropyl, n-butyl, t-butyl and phenyl.

Preferred embodiments of the compound occur when R¹ is a benzyl group and R² is CH₃SCH₂CH₂-; and when R¹ is a benzyl group and R² is C₆H₅SCH₂CH₂-.

The invention is also directed to a fully protected 2-amino-6-halopurine of the formula:

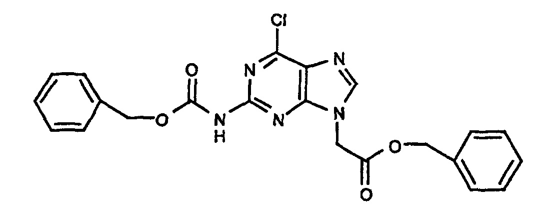

The invention is also directed to a fully protected 2-amino-6-halopurine having the formula:

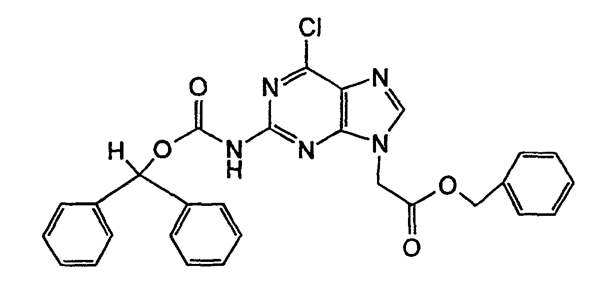

The invention is also directed to a selectively carbamate protected guanine side chain moiety of the general formula: 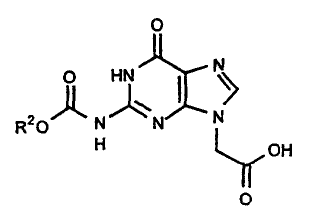 wherein R² is independently selected from the group consisting of methyl, ethyl, 2,2,2-trichloroethyl, 2-(trimethylsilyl)-ethyl, propyl, isopropyl, n-butyl, t-butyl, allyl, 1-isopropyl allyl, cinnamyl, 4-nitrocinnamyl, diphenylmethyl, and a benzyl group or derivative thereof (as defined above).

In another embodiment, R² can be a group having the formula:

R⁵SCH₂CH₂-

wherein R⁵ is selected from the group consisting of methyl, ethyl, propyl, isopropyl, n-butyl, t-butyl and phenyl.

Preferred embodiments of the compound occur when R² is CH₃SCH₂CH₂-, and when R² is C₆H₅SCH₂CH₂-.

The invention is also directed to a selectively carbamate protected guanine side chain moiety having the formula:

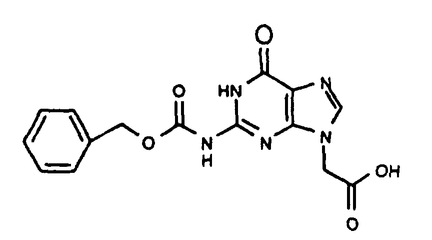

The invention is also directed to the selectively carbamate protected guanine side chain moiety having the formula:

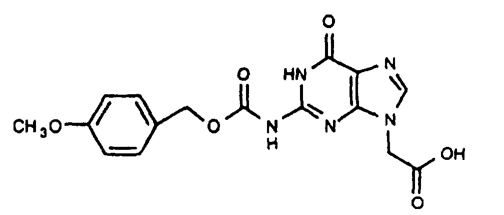

The invention is also directed to a selectively carbamate protected guanine side chain moiety having the formula:

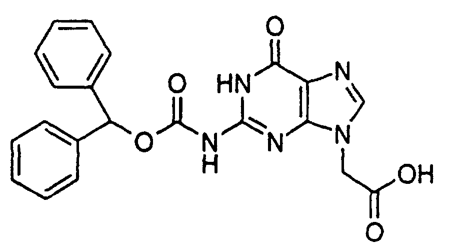

The invention is also directed to a guanine PNA synthon ester of the general formula: 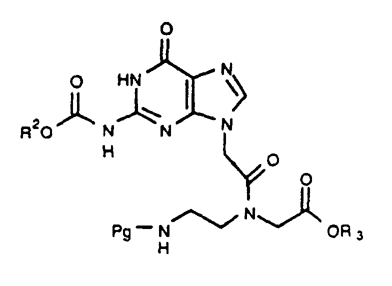 wherein Pg is a protecting group selected from the group consisting of alkyloxycarbonyl, 9-fluorenylmethyloxycarbonyl, 1-methyl-1-(4-biphenyl)- ethyloxycarbonyl, 1-methyl-1 -phenylethyloxy carbonyl, triphenylmethyl, 4-methoxy-triphenylmethyl, and 4,4'-dimethoxytriphenylmethyl; R² is independently selected from the group consisting of methyl, ethyl, 2,2,2-trichloroethyl, 2-(trimethylsilyl)-ethyl, propyl, isopropyl, n-butyl, t-butyl, allyl, 1-isopropyl allyl, cinnamyl, 4-nitrocinnamyl, diphenylmethyl, a benzyl group or derivative thereof (as defined above), and a group having the formula R⁵SCH₂CH₂- (as defined above); and R³ is an alkyl group selected from the group consisting of methyl and ethyl.

The invention is also directed to a guanine PNA synthon ester having the formula:

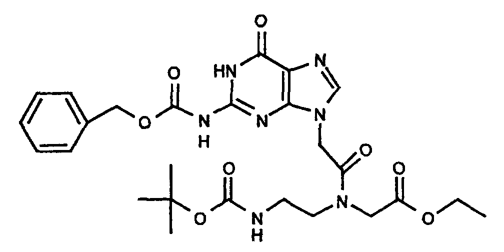

The invention is also directed to a guanine PNA synthon of the general formula: 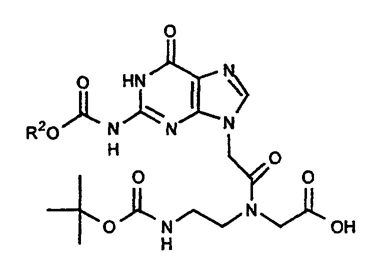 wherein R² is independently selected from the group consisting of methyl, ethyl, 2,2,2-trichloroethyl, 2-(trimethylsilyl)-ethyl, 2-(methylthio)-ethyl, propyl, isopropyl, n-butyl, t-butyl, allyl, 1-isopropyl allyl, cinnamyl, 4-nitrocinnamyl, a benzyl group or derivative thereof (as defined above).

The invention is also directed to the guanine PNA synthon having the formula: 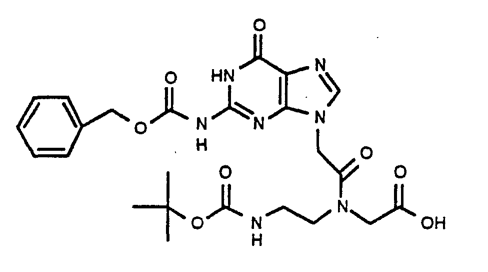 The invention is also directed to the guanine PNA synthon having the formula:

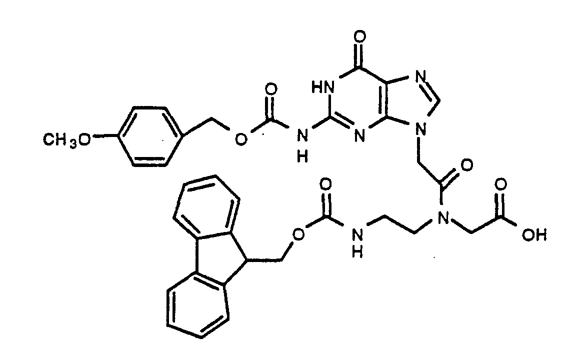

It is an object of this invention to prepare fully protected 2-amino-6-halo purine compounds suitable for conversion to selectively carbamate protected guanine side chain moieties.

It is another object of this invention to prepare selectively carbamate protected guanine side chain moieties.

It is further an object of this invention to prepare novel guanine PNA synthons having selective carbamate protection of the exocyclic 2-amino group.

### Brief Description of the Drawings

Figure I is a schematic representation of the synthesis of the preferred guanine PNA synthon of this invention.

### Detailed Description of the Invention

The applicant has developed a convenient high yield method for preparing novel purine intermediates having selective carbamate protection of the exocyclic 2-amino functional group of the purine heterocycle which are suitable for preparation of novel guanine PNA synthons. Generally, the guanine PNA synthon is assembled by coupling the carbamate protected guanine side chain moiety to a suitably N-protected backbone ester of the amino acid N-(2-aminoethyl)-glycine, followed by hydrolysis of the backbone ester group. The applicants' results are novel in that there is no report of guanine PNA synthon having selective carbamate protection of the exocyclic 2-amino group, wherein the C6 carbonyl group remains unprotected.

### Step 1

With reference to Figure 1, this invention is a method for preparing guanine PNA synthons having selective carbamate protection of the exocyclic 2-amino group. The starting material for preparation of the carbamate protected guanine side chain moiety is a 2-amino-6-halopurine as shown at the far left of Figure 1. Preferably, the 2-amino-6-halopurine is 2-amino-6-chloropurine. The 2-amino-6-halopurine is first alkylated at the N9 nitrogen atom of the purine ring with a protecting moiety thereby forming a partially protected 2-amino-6-halopurine. "Fully protected" is defined as protecting all nucleophilic atoms or functional groups which react with phosgene thereby rendering them inert to phosgene or any phosgene equivalent. Preferably, the partially protected moiety is protected with an acetate ester group.
Thus, in one embodiment, the partially protected 2-amino-6-halopurine compounds of this invention have the general formula: 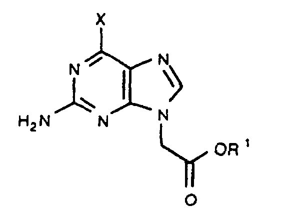 The atom represented by X is a halogen atom selected from the group consisting of F, Cl, Brand I. The alkyl group represented by R¹ is independently selected from the group consisting of methyl, ethyl, 2,2,2-trichloroethyl, 2-(trimethylsilyl)-ethyl, 2(phenylthio)-ethyl, propyl, isopropyl, n-butyl, t-butyl, allyl, 1 -isopropyl allyl, cinnamyl, 4-nitrocinnamyl, and a benzyl group or derivative thereof having the formula: 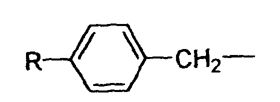 wherein R is hydrogen, isopropyl, t-butyl, or methoxy.

With reference to Figure 1, the most preferred partially protected 2-amino-6-halopurine has the formula:

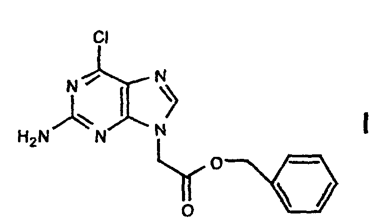

### Step 2

Compound II is the fully protected 2-amino-6-halopurine. With reference to Figure 1, compound I is treated sequentially with triphosgene, diisopropylethylamine (DIEA) and benzyl alcohol. The result is a fully protected 2-amino-6-halopurine (compound II). Conversion of the partially protected 2-amino-6-halopurines to fully protected 2-amino-6-halopurines enables the carbamate protection at a fairly non-nucleophilic exocyclic 2-amino group. Thus, by the method of this invention, a partially protected 2-amino-6-halopurine compound is reacted with at least one (1) molar equivalent of phosgene in the presence of at least two (2) molar equivalents of a non-nucleophilic base thereby generating a 2-isocyanato-6-halopurine compound. Some examples of suitable non-nucleophilic bases are triethylamine, diisopropylethylamine, N-methyl morpholine and N-ethyl morpholine. Preferably the non-nucleophilic base is diisopropylethylamine. The reaction is typically performed in a non-nucleophilic anhydrous solvent which at least partially dissolves the non-nucleophilic base and the partially protected 2-amino-6-halopurine compound. Some examples of suitable solvents are diethyl ether, diisopropylether, dioxane, tetrahydrofuran, acetonitrile, ethyl acetate, dichloromethane, chloroform, carbon tetrachloride, benzene and toluene. Preferably, the solvent is tetrahydrofuran.

Phosgene is a highly toxic gas of chemical formula COCl₂. Because of the inherent dangers associated with handling and accurately dispensing phosgene gas, several phosgene equivalents are available. These include the liquid diphosgene and the solid triphosgene. These equivalents decompose, in situ, to generate two and three equivalents of phosgene, respectively, but are more easily handled and dispensed than is the gaseous equivalent. Consequently, all these reagents are non-limiting examples of phosgene equivalents suitable for the method of this invention.

Preferably, the intermediate 2-isocyanato-6-halopurine compound is not isolated because it is easily hydrolyzed back to the starting partially protected 2-amino-6-halopurine compound during the handling steps required for isolation. Therefore, the 2-isocyanate group of the intermediary 2-isocyanato-6-halopurine compound is then reacted with an alcohol thereby generating a novel fully protected 2-amino-6-halopurine compound having an exocyclic 2-amino group protected as a carbamate.

Suitable alcohols which will react with the exocyclic 2-isocyanato group are methanol, ethanol, 2,2,2-trichloroethanol, 2-(trimethylsilyl)-ethanol, propanol, isopropanol, n-butanol, and t-butanol. Additionally, the alcohol may be an allyl alcohol derivative selected from the group consisting of allyl alcohol, 1-isopropylallyl alcohol, cinnamyl alcohol, and 4-nitrocinnamyl alcohol. Preferably, the alcohol is benzyl alcohol or a derivative thereof having the formula: 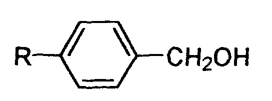 wherein R is hydrogen, isopropyl, t-butyl, or methoxy.

The alcohol also may be diphenylmethanol.

The alcohol also may be a group having the formula:

R⁵SCH₂CH₂OH

wherein R⁵ is selected from the group consisting of methyl, ethyl, propyl, isopropyl, n-butyl, t-butyl and phenyl.

Thus, the fully protected 2-amino-6-halopurine is a compound of the general formula: 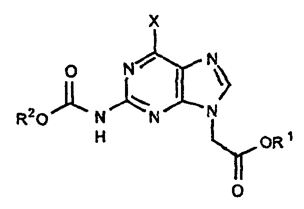 wherein R¹ and R² are defined above. With reference to Figure 1, the preferred fully protected 2-amino-6-halopurine is a compound of formula:

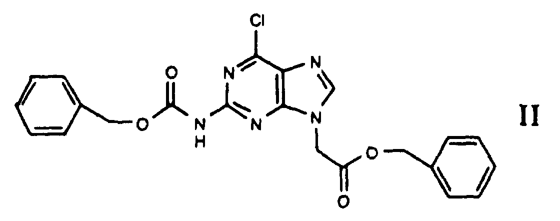

Another preferred fully protected 2-amino-6-halopurine is a compound of the formula

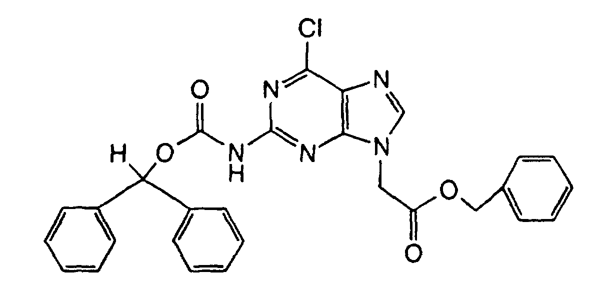

The versatility of this synthetic step is demonstrated by examples of several different fully protected 2-amino-6-chloropurine compounds prepared by simple substitution of a desired alcohol. Thus, Table I summarizes the yield results for several prepared 2-amino-6-chloropurine compounds wherein only the group R² differs. As the table suggests, the yields are moderate and fairly consistent regardless of the composition of the alcohol.

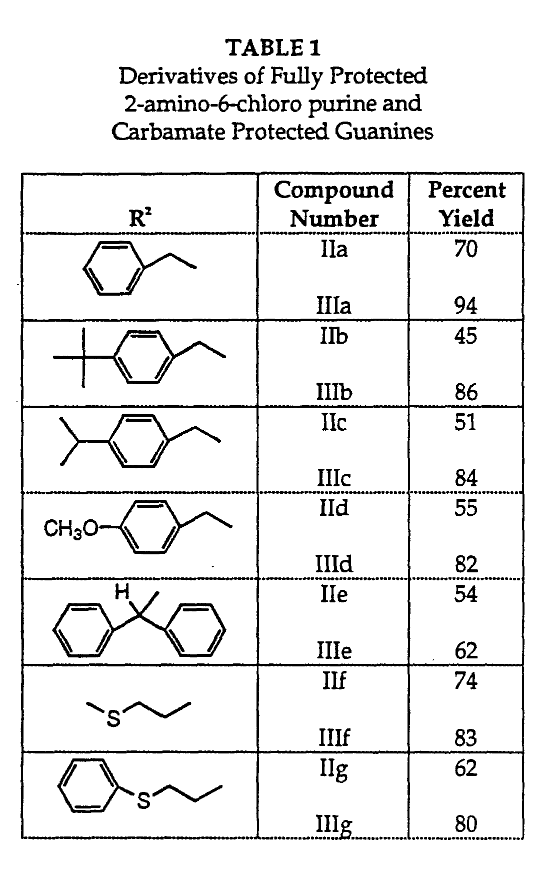

### Step 3

It is known that suitably protected 2-amino-6-halopurine compounds may be converted to protected guanine compounds by several methods involving attack of the 6-halo group with oxygen nucleophiles. See Hodge, et al., Org. Chem. (1990) 56:1553-64. However, this invention discloses a method for converting the 6-halo group to a C6 carbonyl group, and simultaneously hydrolyzing the N⁹ acetate ester group. Thus, carbamate protected guanine side chain moieties are prepared from the fully protected 2-amino-6-halopurine compounds by a method comprising the steps of converting the 6-halo group to a C6 carbonyl group and concurrently hydrolyzing the N⁹ acetate ester group, wherein the alkoxide of 3-hydroxypropionitrile is remarkably utilized to perform-in both transformations.

The alkoxide of 3-hydroxypropionitrile can both convert a 6-halo group to a C6 carbonyl group and hydrolyze the N⁹ acetate ester group. Thus the carbamate protected guanine side chain moiety is easily generated directly from the fully protected 2-amino-6-halopurine in a single reaction. With reference to the preferred embodiment depicted in Figure 1, 2-(N-[benzyloxycarbonyl])-amino-6-chloro-N⁹(benzylcarboxymethyl)-purine (compound II) is directly converted to 2-(N-benzyloxycarbonyl])-N⁹-(carboxymethyl)-guanine (compound III). According to the method, at least three equivalents of the alkoxide of 3-hydroxypropionitrile is reacted with (II). Two equivalents are used to convert the 6-halo group to a C6 carbonyl group and at least one equivalent is simultaneously used to hydrolyze the N⁹ acetate ester group. Consequently, the preferred carbamate protected guanine side chain moiety (III) is prepared in high yield.

According to the method of the invention, at least three (3) equivalents of the alkoxide of 3-hydroxypropionitrile is prepared per equivalent of fully protected 2-amino-6-halopurine by the treatment of 3-hydroxypropionitrile with metal hydride in a non-nucleophilic anhydrous solvent. The metal hydride that may be used include lithium hydride, sodium hydride, potassium hydride and cesium hydride. Examples of suitable non-nucleophilic solvents have been previously described. Preferably, the metal hydride is sodium hydride and the solvent is tetrahydrofuran.

To the prepared solution of alkoxide is added the fully protected 2-amino-6-halopurine compound. The mechanism of this step of the reaction is believed to be as follows. Initially, one equivalent of alkoxide displaces the 6-halo atom thereby forming a 6-cyanoethoxy ether. Thereafter, abstraction of a proton from the 2-cyanoethyl ether group by another equivalent of alkoxide will cause β-elimination thereby forming a C6 carbonyl group, one equivalent of acrylonitrile and one equivalent of 3-hydroxypropionitrile. Thus, two equivalents of alkoxide of 3-hydroxypropionitrile are consumed to convert the 6-halo group to a C6 carbonyl group.

Simultaneous hydrolysis of the N⁹ acetate ester group of the fully protected 2-amino-6-halopurine also comprises reaction with the alkoxide of 3-hydroxypropionitrile. According to the method, one equivalent of the alkoxide of 3-hydroxypropionitrile will react with the N⁹ acetate ester group causing displacement of the original alcohol and thereby forming a 2-cyanoethyl ester and the metal alkoxide of the displaced alcohol. Thereafter, abstraction of a proton from the 2-cyanoethyl ester group by an additional equivalent of alkoxide will cause β-elimination thereby forming the metal salt of the backbone carboxylic acid group, one equivalent of acrylonitrile and one equivalent of alcohol. The alkoxide used to abstract the proton from the cyanoethyl ester may be the alkoxide of the displaced alcohol or a molecule of the alkoxide of 3-hydroxypropionitrile where an excess of the alkoxide of 3-hydroxypropionitrile is used. Thus, only one equivalent of the alkoxide of 3-hydroxypropionitrile is absolutely required to hydrolyze the N⁹ acetate ester group. Preferably, an excess of the alkoxide of 3-hydroxypropionitrile is prepared.

Thus, the products of reacting the fully protected 2-amino-6-halopurine with three (3) equivalents of alkoxide are the metal salt of the selectively carbamate protected guanine side chain moiety, two equivalents of acrylonitrile and one equivalent of 3-hydroxypropionitrile and one equivalent of alcohol which may or may not be 3-hydroxypropionitrile. Thereafter, the metal salt of the selectively carbamate protected guanine side chain moiety is neutralized by treating with at least two equivalents of acid. Neutralization involves protonation of both the N1 nitrogen of the purine heterocycle and protonation of the metal salt of the backbone carboxylic acid group. The acid may be organic or inorganic and should have a pKa of less than two (2) thereby enabling the protonation of both the N1 nitrogen of the purine heterocycle and the backbone carboxylic add group.

In one embodiment of the carbamate protected guanine side chain moiety is a compound of the general formula: 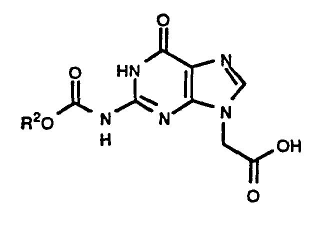 wherein R² is defined above. Preferably, the carbamate protected guanine side chain moiety has the formula:

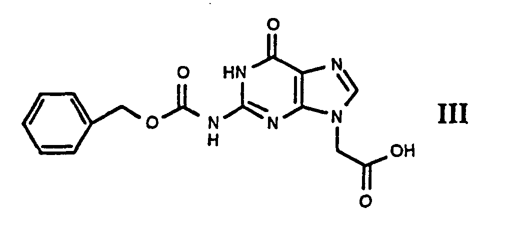

Another preferred selectively carbamate protected guanine side chain moiety has the formula:

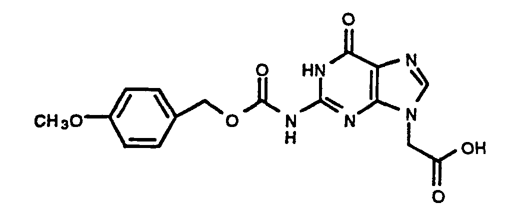

Another preferred selectively carbamate protected guanine side chain moiety has the formula: 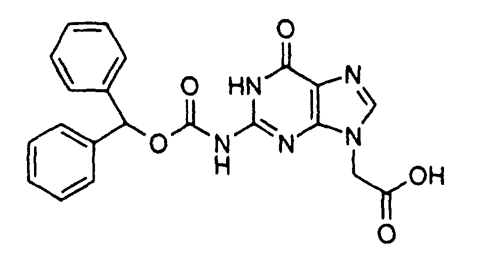 Another preferred selectively carbamate protected guanine side chain moiety has the formula: 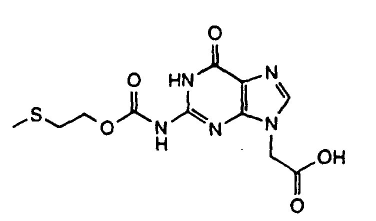 Another preferred selectively carbamate protected guanine side chain moiety has the formula:

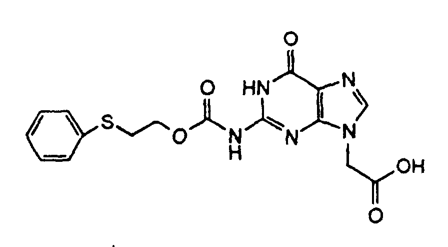

The versatility of this synthetic step is demonstrated by preparation of several different selectively carbamate protected guanine side chain moieties. Thus, Table I summarizes the yield results for several prepared selectively carbamate protected guanine side chain moieties wherein only the alkyl group R² differs. As the table suggests, the yields are excellent and do not substantially differ with the composition of the alkyl group R².

With reference to Figure 1, the final steps in preparing the guanine PNA synthon involve coupling the carbamate protected guanine side chain moiety (III) to a suitably N-protected backbone ester of the amino acid N-(2-aminoethyl)-glycine, followed by hydrolysis of the backbone ester group. These steps generate first the guanine PNA synthon ester (e.g. compound IV), and second the guanine PNA synthon (eg. compound V).

### Step 4

Coupling of the selectively carbamate protected guanine side chain moiety (eg. compound III) to the suitably N-protected backbone ester of the amino acid N-(2-aminoethyl)-glycine, comprises the steps of: first generating the mixed anhydride of the carboxylic acid functional group of the carbamate protected guanine side chain moiety by treatment with a non-nucleophilic base in the presence of a sterically hindered acid chloride, and secondly reacting the prepared mixed anhydride with a suitably N-protected backbone ester of the amino acid N-(2-aminoethyl)-glycine, thereby generating the guanine PNA synthon ester.

The non-nucleophilic base used to generate the mixed anhydride of the selective carbamate protected guanine side chain moiety can be any of the non-nucleophilic bases previously described. Preferably, the base is N-methyl morpholine. The sterically hindered acid chloride is selected from the group consisting of isobutyryl chloride, trimethylacetyl chloride (pivaloyl chloride) and adamantane carboxyl chloride. Preferably, the acid chloride is trimethylacetyl chloride (pivaloyl chloride).
The suitably N-protected backbone ester of the amino acid N-(2-aminoethyl)-glycine, will have the general formula:

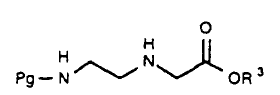

The protecting group represented by Pg is a protecting group which may be selected from the group consisting of alkyloxycarbonyl, 9-fluorenylmethyloxycarbonyl, methyl-1-(4-biphenyl)-ethyloxycarbonyl, 1-methyl-1-phenyl-ethyloxy carbonyl, triphenylmethyl, 4-methoxy-triphenylmethyl, and 4,4'-dimethoxy-triphenylmethyl.
The alkyl group represented by R³ may be selected from the group consisting of methyl and ethyl. Preferably, the protecting group (Pg) is tert-butyloxycarbonyl (t-boc) and the alkyl group (R³) is ethyL Therefore, the preferred guanine PNA synthon ester has the general formula: 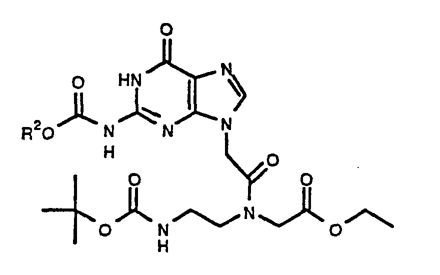 Wherein R² is as defined above.

In the most preferred embodiment, the guanine PNA synthon ester has the formula:

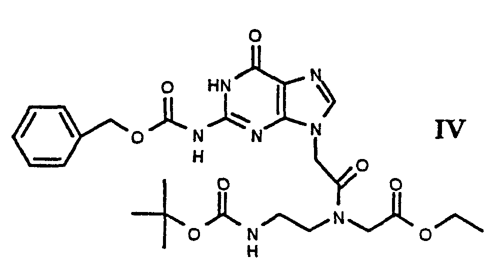

### Step 5

Hydrolysis of the backbone ester group of a guanine PNA synthon ester yields the guanine PNA synthon. Hydrolysis of the backbone ester group comprises the steps of: first reacting the backbone ester group of the guanine PNA synthon ester with a base thereby generating a salt of the carboxylic acid group and then neutralizing the salt of the carboxylic acid with an acid. The base should be strong enough to enable the hydroxide ion to displace the alcohol of the ester. Preferably the base is an inorganic base such as lithium hydroxide, sodium hydroxide or potassium hydroxide.
Hydrolysis of the backbone ester group occurs between -5°C and 50°C but preferably is performed below ambient temperature. The solvent for hydrolysis is a mixture of one or more organic solvents and water containing a ratio between 10 and 90 percent water by volume. Suitable organic solvents are methanol, ethanol, isopropanol and acetonirile. The add used to neutralize the salt of the carboxylic add group may be organic or inorganic and should have a pKa of less than two (2) thereby enabling the protonation of the carboxylic acid group.

In one embodiment of the guanine side PNA synthon is a compound of the general formula: 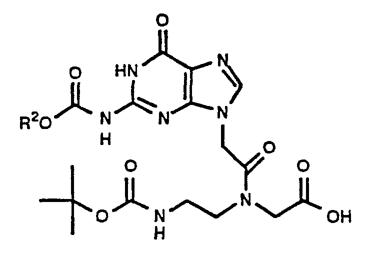 wherein the alkyl group R² is defined above. Preferably, the guanine PNA synthon is a compound of formula:

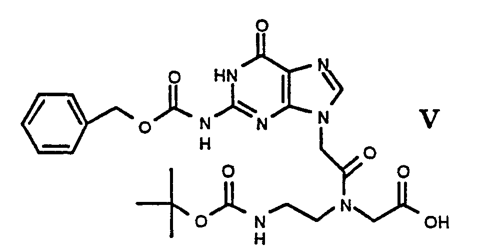

The invention, being generally described above, is now more specifically illustrated by way of the following examples, which are not meant to limit the invention, unless otherwise noted.

### Examples

### Example 1

### Synthesis of 2-amino-6-chloro-N⁹-(benzylcarboxymethyl)-purine (I)

To 6-chloro-2-amino purine (300 g. 1.77 mole; Pharma-Waldorf GmbH, Germany, P/N 471720) and potassium carbonate (366 g; 2.65 mole, Aldrich Chemical, Milwaukee, WI (hereinafter Aldrich) P/N 34,/82-5) was added dimethyl formamide (DMF, 3 L) and the solution was warmed until all the 2-amino-6-chloropurine dissolved (84°C). The mixture was then cooled in an ice bath and benzyl-2-bromoacetate (299 mL, 1.89 mole; Aldrich P/N 24,563-1) was added dropwise over the course of one and one half hours. The mixture was stirred for an additional three hours at 0°C and was then stirred overnight at ambient temperature. The following day the reaction mixture was filtered and the filtrate was then poured into a solution containing 7 liters of water and 150 mL of concentrated hydrochloric acid (HCl). The mixture was stirred for 2 hrs. and the product was then isolated by filtration. The product was washed thoroughly with water and subsequently recrystallized by portion-wise addition of the solid to boiling acetonitrile (3 L). The very red solution was left overnight and filtered the next day. The product was washed thoroughly with methanol and then diethylether. Yield 386 g (69%); ¹H - NMR (d₆ DMSO) δ 8.14 (1 H,s), 7.4-7.3 (5 H, m), 7.02 (2 H,s), 5.21 (2 H,s), 5.08 (2H,s).

### Example 2

### Synthesis of 2-(N-[benzyloxycarbonyl])-amino-6-chloro-N⁹-(benzylcarboxymethyl)purine (IIa)

To 2-amino-6-chloro-N⁹-(benzylcarboxymethyl)-purine (I) (292 g; 0.920 mole) and triphosgene (98.8 g; 0.33 mole; Aldrich P/N 33,075-2) in an oven-dried 5 L three-necked reaction flask under argon standing in an ice bath was added ice cold anhydrous THF (3 L). The mixture was stirred for 15 min. and thereafter diisopropylethylamine (348 mL; 2.0 mole; Lanchaster Synthesis, Windham, NH. (hereinafter Lanchaster) P/N 4310) was added dropwise over a period of 35 min. After stirring for an additional 20 min., benzyl alcohol (153 mL; 1.5 mole; Aldrich P/N 10,800-6) was immediately added. The mixture was allowed to stir overnight while warming to ambient temperature. The reaction mixture was then poured into a solution containing 6.5 L of water and 80 mL of concentrated HCl. The mixture was stirred for 2 hrs. at room temperature and the product was collected by vacuum filtration. The solid was washed thoroughly with water and then recrystallized from denatured ethanol (2 L). The product was collected by vacuum filtration and washed twice with cold ethanol. Yield 293 g (70%); ¹H-NMR (d₆ DMSO) δ 10.86 (1 H,s), 8.52 (1 H,s), 7.5-7.3 (10 H,m), 5.22-5.20 (6 H,m).

### Example 3

### Synthesis of 2-(N-[benzyloxycarbonyl])-N⁹ (carboxymethyl)-guanine (IIIa)

Sodium hydride (18.0 g; 0.75 mole; Aldrich P/N 22,344-1) was added to 1100 mL of dry THF in a dry 5L round-bottom flask blanketed with an argon atmosphere. The reaction flask was placed in a dry-ice acetone bath for a period of 15-20 min. To the solution was added drop wise 51.2 mL (0.75 mole) of 3-hydroxypropionitrile (Aldrich Chemical P/N 10,992-4) over a period of 15-20 minutes. After the addition was complete, the dry-ice acetone bath was removed and stirring was continued while the reaction warmed in an ice bath for 2 hours. Thereafter, the 2-(N-[benzyloxycarbony] amino-6-chloro-N⁹-(benzylcarboxymethyl)-purine (II) (67.9 g 0.15 mole) was added as a dry solid, portion-wise over a period of 10-15 minutes. The ice bath was removed and stirring was continued for another 2 hrs. The total volume was reduced to about 500 mL using reduced pressure evaporation. The resulting mixture was poured into a solution containing 4 L of water and 70 mL of concentrated HCl. The mixture was stirred overnight and then filtered the following day. The solid was washed thoroughly with water and then three times with ethyl acetate. Yield 48.5 g (94%) of a white solid. ¹H-NMR (d₆ DMSO) δ 11.55 (1 H,s),11.38 (1 H,s), 7.95 (1 H,s) 7.5-7.3 (5Hm) 5.26 (2H,s) 4.89 (2H,s).

### Example 4

### Synthesis of 2-(N-[4-(t-butyl)-benzyloxycarbonyl])-amino-6-chloro-N⁹-(benzylcarboxymethyl)-purine (IIb)

To 2-amino-6-chloro-N⁹-(benzylcarboxymethyl)-purine (I) (9.05g; 30 mmol) and triphosgene (2.97 g; 10 mmol; Aldrich P/N 33,075-2) in an oven-dried 500 mL three-necked reaction flask under argon standing in an ice bath was added ice cold anhydrous THF (100 mL). The mixture was stirred for 15 min. and thereafter diisopropylethylamine (1 0.5 mL; 60 mmol; Lanchaster, P/N 431 0) was added drop wise over a period of 5 min. After stirring for an additional 30 minutes, 4-*tert*-butylbenzyl alcohol (8 mL; 45 mmol; Aldrich P/N 18,426-8) was immediately added. The mixture was allowed to stir overnight while warming to ambient temperature. The reaction mixture was then poured into a solution containing 1.0 L of water and 13 mL of concentrated HCl. The mixture was stirred for 30 min. at room temperature and then 250 mL of ethyl acetate was added to partition the product. The layers were separated and the organic layer was washed with 125 mL of 5% aqueous sodium bicarbonate. The organic layer was dried with sodium sulfate, filtered and evaporated. The residue was crystallized from 50 mL of toluene. The product was collected by vacuum filtration.
Yield 6.88 g (45%); ¹H-NMR (CDCl₃) δ 8.04 (1H,s), 7.83 (1H,s), 7.42-7.29 (9H,m), 5.21 (4H,s), 5.01 (2H,s), 1.31 (9H,s).

### Example 5

### Synthesis of 2-(N-[4-(t-butyl)-benzyloxycarbonyl])-N⁹-(carboxymethyl)-guanine (IIIb)

Sodium hydride (1.5 g; 60 mmol; Aldrich P/N 22,344-1) was added to 90 mL of dry THF in a dry 250 mL round-bottom flask blanketed with an argon atmosphere. The reaction flask was placed in a dry-ice acetone bath for a period of 10 min. To the solution was added drop wise 4.1 mL (60 mmol) of 3-hydroxypropionirile (Aldrich Chemical P/N 10,992-4) over a period of 5-10 minutes. After the addition was complete, the dry-ice acetone bath was removed and stirring was continued while the reaction manned in an ice bath for 2 hours. Thereafter, the 2-(N-[t-(butyl)benzyloxycarbonyll)-amino-6-chloro-N⁹-(benzylcarboxymethyl)-purine (IIb) (6.1g; 12 mmol) was added as a dry solid, portion wise over a period of 2-3 minutes. The ice bath was removed and stirring was continued for another 1 hr. Added 200 mL of water and then 9 mL of 6N HCI. The solution was cooled in an ice bath for 30 min. and then the solid was collected by vacuum filtration. The solid was boiled in 50 mL of acetonitrile for 1 hr. and then the solution was cooled to room temperature. The product was collected by vacuum filtration. Yield 4.13 g (86%); ¹H-NMR (d₆ DMSO) δ 11.51 (1 H,s), 11.38 (1 H,s), 7.94 (1 H,s), 7.44-7.33 (4 H, dd), 5.22 (2 H,s) 4.88 (2 H,s), 1.28 (9 H,s).

### Example 6

### Synthesis of 2-(N-[4-(isopropyl)-benzyloxycarbonyl])-amino-6-chloro-N⁹-(benzylcarboxymethyl)-purine (IIc)

To 2-amino-6-chloro-N⁹-benzylcarboxymethyl)-purine (I) (9.05 g; 30 mmol) and triphosgene (2.97 & 10 mmol; Aldrich P/N 33,075-2) in an oven-dried 500 mL three necked reaction flask under argon standing in an ice bath was added ice cold anhydrous THF (100 mL). The mixture was stirred for 15 min. and thereafter diisopropylethylamine (10.5 ml, 60 mmol; Lanchaster, P/N 4310) was added drop wise over a period of 5-10 min. After stirring for an additional 30 minutes, 4-isopropylbenzyl alcohol (6.9 mL; 45 mmol; Aldrich P/N 19,603-7) was immediately added. The mixture was allowed to stir overnight while warming to ambient temperature. The reaction mixture was then poured into a solution containing 1.0 L of water and 13 mL of concentrated HCl. The mixture was stirred for 30 min. at room temperature and then 250 mL of ethyl acetate was added to partition the product. The layers were separated and the organic layer was washed with 125 mL of 5% aqueous sodium bicarbonate. The organic layer was dried with sodium sulfate, filtered and evaporated. The residue was crystallized from 25 mL of toluene. The product was collected by vacuum filtration. Yield 7.50g (51%); ¹H-NMR (CDCl₃) δ.04 (1H,s), 7.74 (1H,s), 7.35-7.20 (9H,m), 5.22 (4 H,m), 5.02 (2 H,s), 2.96-2.84 (1 H, septet), 1.25 (6 H,d).

### Example 7

### Synthesis of 2-(N-[4-(isopropyl)-benzyloxycarbonyl)-N⁹-(carboxymethyl)-guanine (IIIc)

Sodium hydride (1.5 g; 60 mmol; Aldrich P/N 22,344-1) was added to 90 mL of dry THF in a dry 250 mL round-bottom flask blanketed with an argon atmosphere. The reaction flask was placed in a dry-ice acetone bath for a period of 10 min. To the solution was added dropwise 4.1 mL (60 mmol) of 3-hydroxypropionitrile (Aldrich Chemical P/N 10,992-4) over a period of 1-2 minutes. After the addition was complete, the dry-ice acetone bath was removed and stirring was continued while the reaction warmed in an ice bath for 2 hours. Thereafter, the 2-(N-[4-(isopropyl)benzyloxycarbonyl])-amino-6-chloro-N⁹-(benzylcarboxymethyl)-purine (IIc) (6.1g; 12 mmol) was added as a dry solid, portion-wise over a period of 2-3 minutes. The ice bath was removed and stirring was continued for another 1 hr. Added 300 mL of water and then 11 mL of 6N HCl. The solution was cooled in an ice bath for 30 min. and then the solid was collected by vacuum filtration. The solid was boiled in 50 mL of acetonirile for 1 hr. and then the solution was cooled to room temperature. The product was collected by vacuum filtration. Yield 4.84 g (84 %); ¹H-NMR (d₆ DMSO) δ 11.51 (1 H,s), 11.38 (1 H,s), 8.18 (1 H,s) 7.38-7.24 (4H,dd) 5.21 (2H,s) 4.87 (2H,s), 2.95-2.80 (1H,septet), 1 .2 (6H,d).

### Example 8

### Synthesis of 2-(N-[4-(methoxy)-benzyloxycarbonyl])-amino-chloro-N⁹-(benzylcarboxymethyl)-purine (IId)

To 2-amino-6-chloro-N⁹-(benzylcarboxymethyl)-purine (1) (20 g; 63 mmol) and triphosgene (7.46 g; 25 mmol; Aldrich P/N 33,075-2) in an oven-dried 1 L three-necked reaction flask under argon standing in an ice bath was added ice cold anhydrous THF (189 mL). The mixture was stirred for 20 min. and thereafter diisopropylethylamine (24.1 mL; 140 mmol; Lanchaster, P / N 4310) was added dropwise over a period of 5-10 min. After stirring for an additional 30 minutes, 4-methoxybenzyl alcohol (9.9 mL; 79 mmol; Aldrich P/N 13,690-5) was immediately added. The mixture was allowed to stir overnight while warming to ambient temperature. The reaction mixture was then poured into a solution containing 200 mL of water and 4.7 mL of concentrated HCl. The mixture was stirred for 2 hrs. at room temperature and the product was collected by vacuum filtration. The solid was washed thoroughly with water and then recrystallized from acetonitrile (500 mL). The product was collected by vacuum filtration and washed twice with cold acetonitrile. Yield 16.2 g (55%); ¹H-NMR (CDCl₁₃) δ 10.76 (1H,s), 8.5 (1 H,s), 7.4-6.8 (9 H,m), 5.2-5.1(6 H,m), 3.75(3 H,s).

### Example 9

### Synthesis of 2-(N-[4-(methoxy)benzyloxycarbonyl])-N⁹-(carboxymethyl)-guanine (IIId)

Sodium hydride (3.9 g; 160 mmol; Aldrich P/N 22,344-1) was added to 200 mL of dry THF in a dry 1 L round-bottom flask blanketed with an argon atmosphere. The reaction flask was placed in a dry-ice acetone bath for a period of 15-20 min. To the solution was added dropwise 10.95 mL (160 mmol) of 3-hydroxypropionitrile (Aldrich Chemical. P1 N 10,992-4) over a period of 15-20 minutes. After the addition was complete, the dry-ice acetone bath was removed and stirring was continued while the reaction warmed in an ice bath for 2 hours. Thereafter, the 2-(N-[4-(methoxy-benzyloxy carbony 1])-amino-6-chloro-N⁹-(benzylcarboxymethyl)-purine (IId) (15 g; 32.2 mmol) was added as a dry solid, portion-wise over a period of 10-15 minutes. The ice bath was removed and stirring was continued for another 2 hrs. The total volume was reduced to about 70 mL using reduced pressure evaporation. The resulting mixture was poured into a solution containing 200 mL of water and 21 mL of concentrated HCl The mixture was stirred overnight and then filtered the following day. The solid was washed thoroughly with water and then three times with ethyl acetate. Yield 9.9 g (82%)¹H-NMR (d₆ DMSO) δ 11.40 (1H,s), 7.95 (1H,s), 7.4-7.3 (2H,d) 7.0-6.9 (2H,d) 5.15 (2H,s) 4.85 (2H,s), 3.8 (3H,s).

### Example 10

### Synthesis of N-([2-[N-benzyloxycarbonyl]-guanine-9-y-acetyl{N-(2-[t-butyloxycarbonyl]-2-aminoethyl)-glycine ethyl ester (IV)

To 136 g (396 mmole) of 2-(N-[benzyloxycarbonyl])-N⁹-(carboxymethyl)-guanine (III) was added 1600 mL of dry dimethylformamide (DMF). The solution was stirred for 30 min. while cooling in an ice bath. Trimethylacetyl chloride 97.6 mL (792 mmole) (Aldrich, P/ N T7,260-5) was added and the reaction stirred for an additional 10 min. N-methyl morpholine (218 mL; 1.98 mole; Fluka Chemical Ronkonkoma, NY, P/N 67870) was added dropwise over 20-30 minutes and then the reaction stirred for another 30 minutes. To the stirring solution was added 117 g (475 mmole) of *N*¹-(*tert*-butyloxycarbonyl)-N⁴-(2-aminoethyl)-glycine ethyl ester (Millipore Corporation, P/N GEN PNABKB) dissolved in 100 mL of dry DMF. The reaction was stirred overnight while warming to room temperature. The insoluble N-methyl morpholine hydrochloride was filtered off and the cake was washed with acetonitrile. To the filtrate was added 2.5 mL of methanol and then the filtrate was completely evaporated to a tan solid. The solid was recrystallized from 3 L of solvent containing 1/1/1 ethanol/acetonitrile/water. Yield 178.2g(311 mmole; 78%) Due to the limited rotation around the secondary amide bond, several of the proton signals are split in the ratio of 2:1, wherein the rotomers are distinguishable on the NMR time scale. Thus, several signals corresponding to the major rotomer component are designated by "mj" whereas the minor rotomer component is designated by "mi." ¹H NMR (DMSO) δ 11.44 (1 H, d), 11.34 (1 H, s), 7.80 (1 H, m), 7.5-7.3 (5 H, m), 6.98 (1 H; mj, t), 6.73 (1 H; mi, m), 5.26 (2 H, s), 5.09 (2H; mj, s), 4.92 (2H; mi, s), 4.41 (2H; mi, s), 4.06 (2H; mj, s), 4.25-4.15 (2H; mi, q), 4.13 4.03 (2H; mj, q), 3.60-2.95 (4H, mm), 1.35 (9H, s), 1.3-1.1 (3H, dt).

### Example 11

### Synthesis of N-([2-[N-benzyloxycarbonyl]-guanine-9-yl-acetyl]-N-(2-)[t-butyloxycarbonyl]-aminoethyl)-glycine (V)

To 183.3 g (320 mmol) of N-([2-[N-benzyloxycarbonyl)-guanine-9-yl]-acetyl)-N(2-(t-butyloxycarbonyl)-2-aminoethyl)-glycine ethyl ester (IV) was added 960 mL of ethanol (denatured; VWR Scientific, P/N VWO470), 960 mL of acetonitrile and 480 mL of water. The solution was heated at reflux until all the solid was dissolved. The solution was cooled to ambient temperature and then to less than 5°C in an ice/salt bath. To the briskly stirring suspension was added rapidly, 1.6 L of cold (<5 °C) 2N lithium hydroxide solution (134.3 g LiOH, VWR Scientific, P/N JTP406 dissolved and diluted to 1.6 L with water). After stirring exactly five minutes, the reaction was quenched by the addition of 1.54 L of cold (<5 °C) aqueous 2N HCl. (The pH of the reaction was 4.5 and the temperature about 16 °C after the addition.) The solution was filtered through a course flitted glass scinter to remove any insoluble material and then cooled to <10 °C. To the briskly stirring solution was then added 160 mL of aqueous 2N HCl dropwise over 30 minutes (pH = 2-3 by paper). An additional 11 mL of aqueous 3N HCl was then added dropwise to finally adjust the pH to 1-2 by paper. The-solution then stirred in an ice bath for 3 more hrs. and the product was collected by vacuum filtration. The solid was washed liberally with water. Yield 173.6 g (319 mmol; 99%) ¹H-NMR (d₆ DMSO) δ 11.5 (1 H, d), 11.38 (1 H, s), 7.88 (1 H, m), 7.5-7.3 (5H, m), 7.01 (1 H,; mj, m), 6.76 (1 H;-mi, m), 5.26 (2H, s), 5.09 (2H; mj, s), 4.94 (2H; mi, s), 4.32 (2H; mi, s), 3.99 (2H; mj, s), 3.5-2.9 (4H, mm), 1.35 (9H, s).

### Example 12

### Synthesis of 2-(N-[benzhydroloxycarbonyl])-amino-6-chloro-N⁹-(benzylcarboxymethyl)-purine (IIe)

To 62 mmol of 2-amino-6 chloro-N⁹-benzylcarboxymethyl-purine was added about 190 mL of freshly distilled, ice cold, tetrahydrofuran. While the solution stirred in an ice bath, 22.4 mmol of triphosgene was added. The reaction was allowed to stir 1 hr. at 0°C and then 136 mmol of diisopropylethylamine was added dropwise. After stirring 30 minutes at 0°C, 74.4 mmol of benzhydrol alcohol was added. The reaction was allowed to stir overnight while warming to room temperature. In the morning ethanol was added and the reaction was concentrated to dryness. The residue was partitioned in dichloromethane and an aqueous solution of 10% citric acid. The layers were separated and washed 1x with aqueous 5% sodium bicarbonate solution. The dichloromethane layer was dried, filtered and evaporated. The product was recrystallized form methanol. Yield 54%
¹HNMR (d₆DMSO) d 11.0 (1H, s), 8.5 (1H,s), 7.6-7.2 (15H, m), 6.8 (1H, s), 5.15 (4H,m)

### Example 13

### Synthesis of 2-(N-[benzhydryloxycarbonyl])-N⁹-(carboxymethyl)-guanine (IIIe)

To 365 mmol of 95% sodium hydride was added about 450 mL of freshly distilled tetrahydrofuran. The solution was cooled in an dry ice/acetone bath for 20 minutes (-78°C) and then 365 mmol of 3-hydroxypropionitrile was added. Reaction was stirred at 0°C for 2.5 hours and then 73 mmol of 2-(N-[benzhydroloxycarbonyl])-amino-6-chloro-N⁹-(benzylcarboxymethyl)-purine (IIe) was added. The reaction was allowed to stir overnight while warming to room temperature. In the morning, about 300 mL of the solvent was evaporated and then the residue was poured into a solution containing 800 mL water and then acidified by the addition of a 20% aqueous citric acid solution until the pH was about 3-4. The product was collected by vacuum filtration and then crystallized from methanol. Yield 62%.
¹HNMR (d₆DMSO) d 11.7 (1H, s), 11.2 (1H, s), 8.95 (1H, s), 7.6-7.2 (10H, m), 6.85 (1H, s) 5.9 (4H, s)

### Example 14

### Synthesis of 2-(N-[2-(methylthio)-ethyloxycarbonyl])-amino-6-chloro-N⁹-(benzylcarboxy-methyl)-purine (IIf)

To 50 mmol of 2-amino-6 chloro-N⁹-benzylcarboxymethyl-purine was added about 200 mL of freshly distilled tetrahydrofuran. The reaction was cooled for 20 minutes in an ice bath and then 20 mmol of triphosgene was added. The reaction was allowed to stir 30 minutes at 0°C and then 130 mmol of diisopropylethylamine was added dropwise. After stirring 20 minutes at 0°C, 70 mmol of 2-(methylthio)-ethanol was added. The reaction was allowed to stir overnight while warming to room temperature. In the morning, the reaction was concentrated to about half volume and then poured into a stirring solution containing 500 mL of water and 30 mmol of HCl. This mixture was allowed to stir for 30 minutes and then the product was then collected by vacuum filtration. The product was recrystallized from ethanol. Yield 74%
¹HNMR (d₆DMSO) d 10.8 (1H, s), 8.5 (1H, s), 7.35 (5H, m), 5.22 (4H, m), 4.25 (2H,t), 2.75 (2H,t), 2.15 (3H,s)

### Example 15

### Synthesis of 2-(N-[2-(methylthio)-ethyloxycarbonyl])-N⁹-(carboxymethyl)-guanine (IIIf)

To 75 mmol of 95% sodium hydride was added about 100 mL of freshly distilled tetrahydrofuran. The solution was cooled in an ice bath for 20 minutes and then 75 mmol of 3-hydroxypropionitrile was added. Reaction was stirred at 0°C for 2 hours and then 15 mmol of 2-(N-[2-(methylthio)-ethyloxycarbonyl])-amino-6-chloro-N⁹-(benzylcarboxy-methyl)-purine (Iif) was added. The reaction was allowed to stir overnight while warming to room temperature. In the morning, the solvent was completely evaporated and then a solution containing 200 mL of water, 54 grams of sodium chloride and 8 grams of K₂S₂O₇ was added. The solution was stirred briskly for 15 minutes and then the solid product filtered off. The product as purified by boiling in acetonitrile. Yield 83%.
¹HNMR (d₆DMSO) d 11.52 (1H, s), 11.37 (1H, s) 7.93 (1H, s), 4.9 (2H, s), 4.35 (2H,t), 2.785 (2H,t), 2.15 (3H,s)

The 2-(N-[2-(phenylthio)-ethyloxycarbonyl])-amino-6-chloro-N⁹-(benzylcarboxymethyl)-purine (IIg) and 2-(N-[2-(phenylthio)-ethyloxycarbonyl])-N⁹-(carboxymethyl)-guanine (IIIg) compounds were prepared by methods similar to those described above.

## Claims

1. A method for synthesizing a guanine PNA synthon or its ester of the formula: 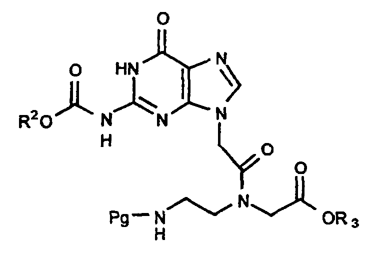
wherein Pg is a protecting group selected from the group consisting of alkyloxycarbonyl, 9-fluorenylmethyloxycarbonyl, 1-methyl-1-(4-biphenyl)ethyloxycarbonyl, 1-methyl-1-phenyl-ethyloxy carbonyl, triphenylmethyl, 4-methoxytriphenylmethyl, and 4,4'-dimethoxy-triphenylmethyl;
R² is selected from the group consisting of methyl, ethyl, 2,2,2-trichloroethyl, 2-(trimethylsilyl)-ethyl, propyl, isopropyl, n-butyl, t-butyl, allyl, 1-isopropyl allyl, cinnamyl, 4-nitrocinnamyl, diphenylmethyl,
a benzyl group or derivative thereof having the formula: 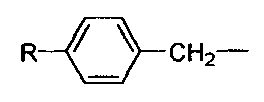
wherein R is hydrogen, isopropyl, t-butyl, or methoxy, and a group having the formula:
R⁵SCH₂CH₂-
wherein R⁵ is selected from the group consisting of methyl, ethyl, propyl, isopropyl, n-butyl, t-butyl and phenyl, and
R₃ is selected from the group consisting of hydrogen, methyl and ethyl; the method comprising the steps of:
(i) synthesizing a carbamate protected guanine side chain moiety of the formula: 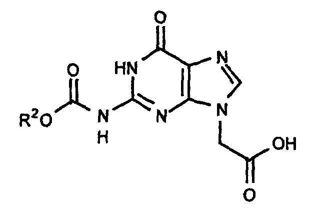
wherein R² is as defined above;
(ii) coupling said carbamate protected guanine side chain moiety to an N-protected backbone ester of the amino acid N-(2-aminoethyl)-glycine of the formula: 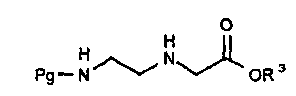
wherein Pg is as defined above; and
R³ is selected from the group consisting of methyl and ethyl,
thereby forming said guanine PNA synthon ester; and
(iii) optionally hydrolyzing the backbone ester group of said guanine PNA synthon ester thereby forming said guanine PNA synthon.

2. The method of claim 1 where the carbamate protected guanine side chain moiety of the formula: 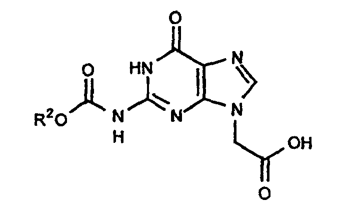
wherein R² is as defined in claim 1,
is prepared by a method comprising the steps of:
(i) reacting the exocyclic 2-amino group of a partially protected 2-amino-6-halopurine compound of the formula: 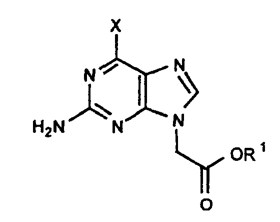 wherein
X is a halogen atom, and
R¹ is selected from the group consisting of methyl, ethyl, 2,2,2-trichloroethyl, 2-(trimethylsilyl)-ethyl, 2-(phenylthio)-ethyl, propyl, isopropyl, n-butyl, t-butyl, allyl, 1-isopropyl allyl, cinnamyl, 4-nitrocinnamyl, and a benzyl group or derivative thereof having the formula: 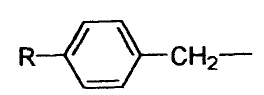 wherein
R is hydrogen, isopropyl, t-butyl, or methoxy; with at least one equivalent of phosgene in the presence of at least two equivalents of non-nucleophilic base, thereby generating a 2-isocyanato-6-halopurine compound;
(ii) reacting said 2-isocyanato-6-halopurine compound with an alcohol, thereby generating a fully protected 2-amino-6-halopurine compound of the formula: 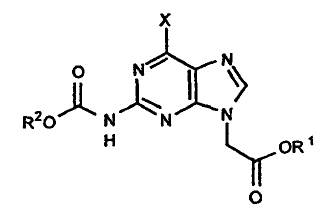 wherein
X and R¹ are as defined above, and
R² is as defined in claim 1;
(iii) reacting said fully protected 2-amino-6-halopurine compound with at least three (3) equivalents of a metal alkoxide of 3-hydroxypropionitrile in an anhydrous non-nucleophilic solvent, thereby generating a metal salt of a carbamate protected guanine side chain moiety; and
(iv) neutralizing said metal salt of said carbamate protected guanine side chain moiety with an acid under conditions suitable for protonation of both the N1 nitrogen atom of the purine heterocycle and the backbone carboxylic acid functional group, thereby forming said carbamate protected guanine side chain moiety.

3. The method of claim 2 wherein the alcohol is selected from the group consisting of methanol, ethanol, 2,2,2-trichloroethanol, 2-(trimethylsilyl)-ethanol, n-propanol, isopropanol, n-butanol, t-butanol, allyl alcohol, 1-isopropyl allyl alcohol, cinnamyl alcohol, 4-nitrocinnamyl alcohol, diphenylmethanol,
a benzyl alcohol or derivative thereof having the formula: 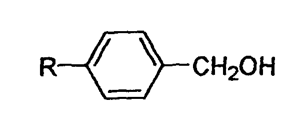
wherein R is hydrogen, isopropyl, t-butyl, or methoxy, and a compound having the formula:
R⁵SCH₂CH₂OH
wherein R⁵ is selected from the group consisting of methyl, ethyl, propyl, isopropyl, n-butyl, t-butyl and phenyl.

4. The method of claim 2 wherein the partially protected 2-amino-6 halopurine has the formula:

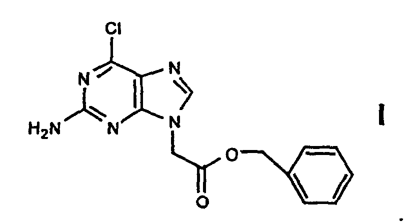

5. The method of claim 2 wherein the carbamate protected guanine side chain moiety is selected from the group consisting of:
(a) a compound having the formula: 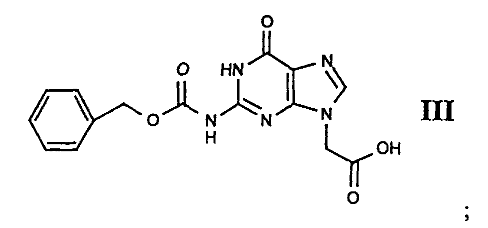
(b) a compound having the formula: 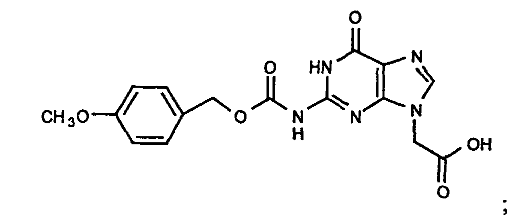 and
(c) a compound having the formula:

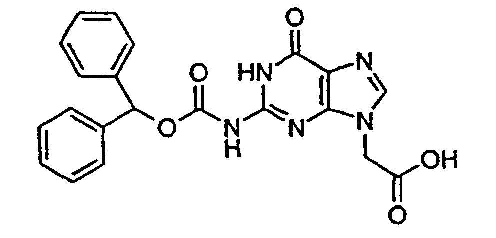

6. The method of claim 1 wherein coupling said protected guanine side chain moiety to an N-protected backbone ester of the amino acid N-(2-aminoethyl)glycine comprises the steps of:
(a) forming a mixed anhydride of said carbamate protected guanine side chain moiety; and
(b) reacting said mixed anhydride with said N-protected backbone ester of the amino acid N-(2-aminoethyl)-glycine, thereby forming said guanine PNA synthon ester.

7. A fully protected 2-amino-6-halopurine compound of the formula: 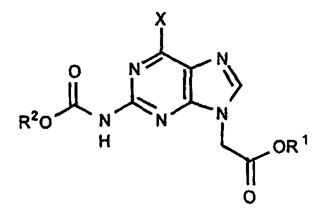
wherein X and R¹ are as defined in claim 2, and
R² is as defined in claim 1;
and said fully protected 2-amino-6-halopurine compound having the above formula:
(i) wherein R¹ is a benzyl group and R² is CH₃SCH₂CH₂-, or
(ii) wherein R¹ is a benzyl group and R² is C₆H₅SCH₂CH₂-; and said fully protected 2-amino-6-halpurine compound having the formula: 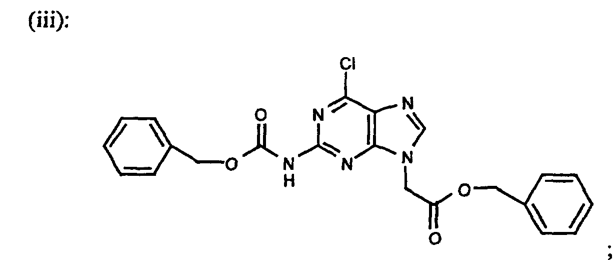 or

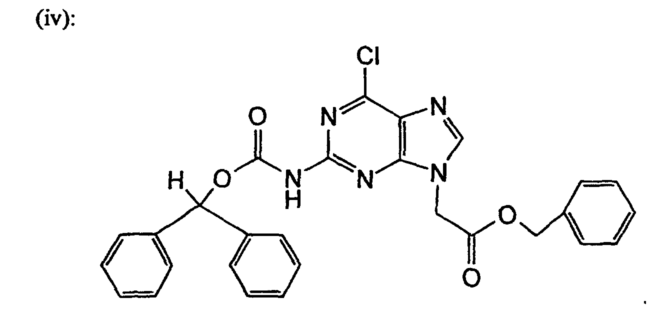

8. A carbamate protected guanine side chain moiety of the formula: 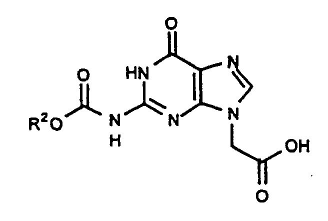
wherein R² is as defined in claim 1;
and said carbamate protected guanine side chain moiety having the formula: 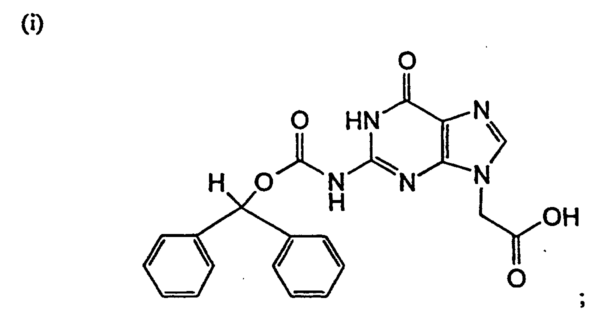 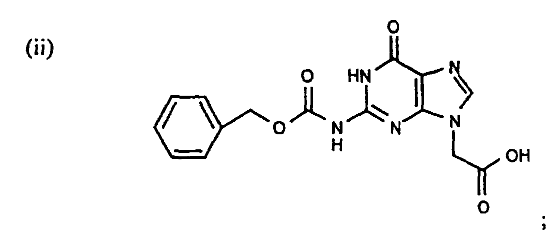 or

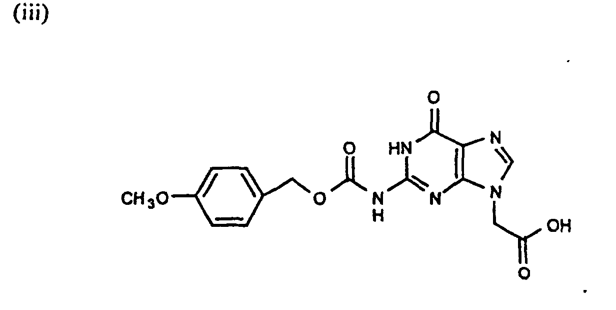

9. A guanine PNA synthon or its ester of the formula: 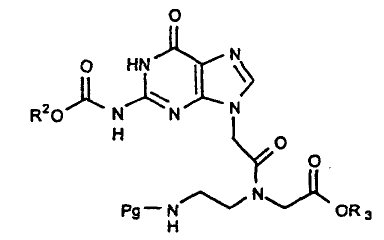
wherein Pg and R² are as defined in claim 1; and
R₃ is selected from the group consisting of hydrogen, methyl and ethyl;
and said guanine PNA synthon or its ester having the formula: 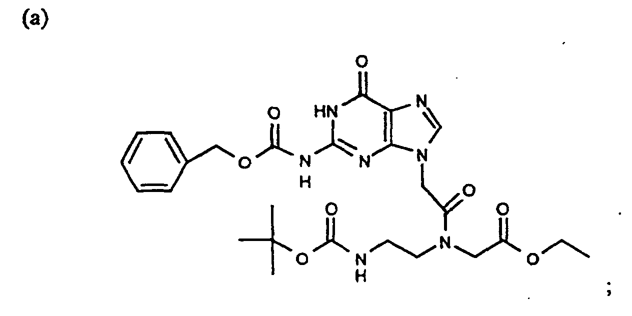 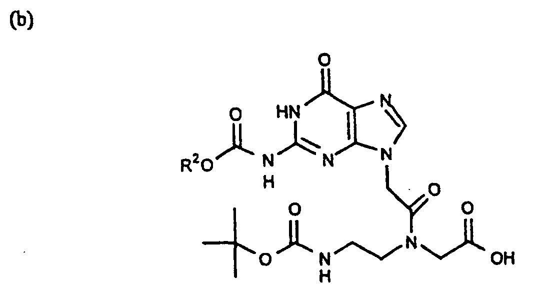
wherein R² is as defined in claim 1; 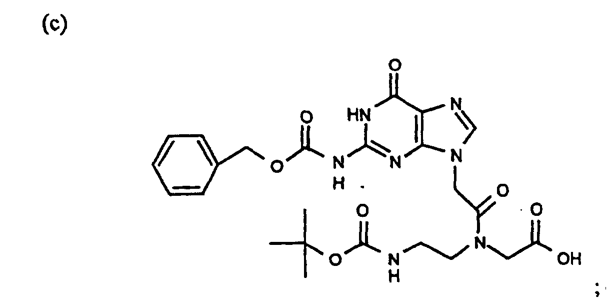 or

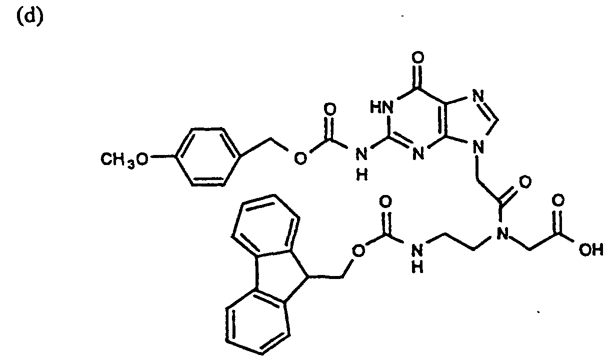

## Patentansprüche

1. Ein Verfahren zur Synthese eines Guanin-PNA-Synthons oder dessen Ester der Formel 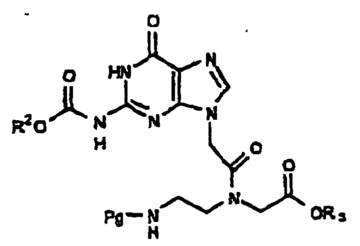 worin
Pg eine Schutzgruppe ist, die aus der Gruppe bestehend aus Alkyloxycarbonyl, 9-Fluorenylmethyloxycarbonyl, 1-Methyl-1-(4-biphenyl)ethyloxycarbonyl, 1-Methyl-1-phenylethyloxycarbony), Triphenylmethyl, 4-Methoxytriphenylmethyl und 4,4'-Dimethoxytriphenylmethyl ausgewählt ist;
R² aus der Gruppe bestehend aus einer Methyl-, Ethyl-, 2,2,2-Trichlorethyl-, 2-(Trimethylsilyl)ethyl-, Propyl-, Isopropyl-, n-Butyl-, t-Butyl-, Allyl-, 1-Isopropylallyl-, Cinnamyl 4-Nitrocinnamyl-, Diphenylmethylgruppe,
einer Benzylgruppe oder einem Derivat davon der Formel 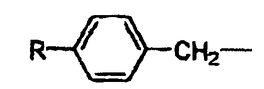
worin R ein Wasserstoffatom, eine Isopropyl-, t-Butyl- oder Methoxygruppe ist, und einer Gruppe der Formel
R⁵SCH₂CH₂-
worin R⁵ aus der Gruppe bestehend aus einer Methyl-, Ethyl-, Propyl-, Isopropyl-, n-Butyl-, t-Butyl- und Phenylgruppe ausgewählt ist; und
R₃ aus der Gruppe bestehend aus einem Wasserstoffatom, einer Methyl- und einer Ethylgruppe ausgewählt ist,
wobei das Verfahren die Schritte umfasst:
(i) Synthetisieren einer Carbamat-geschützten Guanin-Seitenketteneinheit der Formel 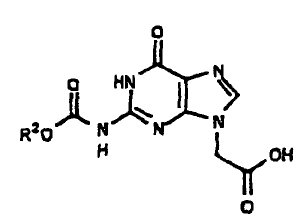
worin R² die vorstehend angegebene Bedeutung hat,
(ii) Koppeln der Carbamat-geschützten Guanin-Seitenketteneinheit mit einem N-geschützten Ester-Grundgerüst der Aminosäure N-(2-Aminoethyl)-glycin der Formel 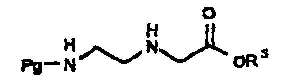
worin Pg die vorstehend angegebene Bedeutung hat, und
worin R₃ aus der Gruppe bestehend aus einer Methyl- und einer Ethylgruppe ausgewählt ist, wodurch der Guanin-PNA-Synthonester gebildet wird, und
(iii) gegebenenfalls Hydrolysieren der Ester-Grundgerüstgruppe des Guanin-PNA-Synthonesters, wodurch das Guanin-PNA-Synthon gebildet wird.

2. Verfahren nach Anspruch 1, bei dem die Carbamat-geschützte Guanin-Seitenketteneinheit der Formel 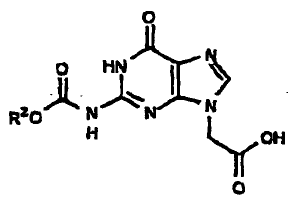
worin R² die in Anspruch 1 angegebene Bedeutung hat,
durch ein Verfahren hergestellt wird, das die Schritte umfasst:
(i) Umsetzen der exocyclischen 2-Aminogruppe einer partiell geschützten 2-Amino-6-halopurinverbindung der Formel 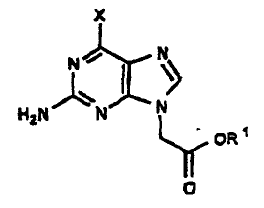 worin
X ein Halogenatom ist, und
R¹ aus der Gruppe bestehend aus einer Methyl-, Ethyl-, 2,2,2-Trichlorethyl-, 2-(Trimethylsilyl)ethyl-, 2-(Phenylthio)ethyl-, Propyl-, Isopropyl-, n-Butyt-, t-Butyl-, Allyl-, 1-Isopropylallyl-, Cinnamyl-, 4-Nitrocinnamylgruppe und einer Benzylgruppe oder einem Derivat davon der Formel 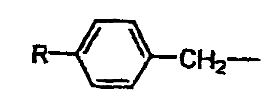
worin R ein Wasserstoffatom, eine Isopropyl-, t-Butyl- oder Methoxygruppe ist, ausgewählt ist,
mit mindestens einem Äquivalent Phosgen in Gegenwart von mindestens zwei Äquivalenten einer nicht-nucleophilen Base, wodurch eine 2-Isocyanato-6-halopurinverbindung erzeugt wird;
(ii) Umsetzen der 2-Isocyanato-6-halopurinverbindung mit einem Alkohol, wodurch eine vollständig geschützte 2-Amino-6-halopurinverbindung der Formel 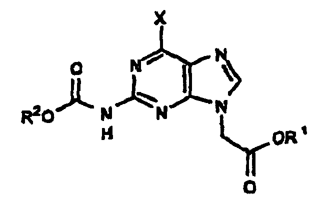 erzeugt wird, worin X und R¹ die vorstehend angegebene Bedeutung haben, und
R² die in Anspruch 1 angegebene Bedeutung hat;
(iii) Umsetzen der vollständig geschützten 2-Amino-6-halopurinverbindung mit mindestens drei (3) Äquivalenten eines Metallalkoxids von 3-Hydroxypropionitril in einem wasserfreien, nicht-nucleophilen Lösungsmittel, wodurch ein Metallsalz einer Carbamat-geschützten Guanin-Seitenketteneinheit erzeugt wird; und
(iv) Neutralisieren des Metallsalzes der Carbamat-geschützten Guanin-Seitenketteneinheit mit einer Säure unter Bedingungen, die zur Protonierung von sowohl dem N1-Stickstoffatom des Purin-Heterocyclus als auch der funktionellen Carbonsäuregruppe des Grundgerüsts geeignet sind, wodurch die Carbamat-geschützte Guanin-Seltenketteneinheit gebildet wird.

3. Verfahren nach Anspruch 2, bei dem der Alkohol aus der Gruppe bestehend aus Methanol, Ethanol, 2,2,2-Trichlorethanol, 2-(Trimethylsilyl)ethanol, n-Propanol, Isopropanol, n-Butanol, t-Butanol, Allylalkohol, 1-Isopropylallylalkohol, Zimtalkohol, 4-Nitrozimtatkohol, Diphenylmethanol,
einem Benzylalkohol oder einem Derivat davon der Formel 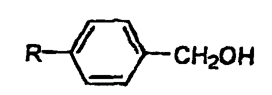
worin R ein Wasserstoffatom, eine Isopropyl-, t-Butyl- oder Methoxygruppe ist, und einer Verbindung der Formel
R⁵SCH₂CH₂OH
ausgewählt ist, worin R⁵ aus der Gruppe bestehend aus einer Methyl-, Ethyl-, Propyl-, Isopropyl-, n-Butyl-, t-Butyl- und Phenylgruppe ausgewählt ist.

4. Verfahren nach Anspruch 2, bei dem das partiell geschützte 2-Amino-6-halopurin die Formel 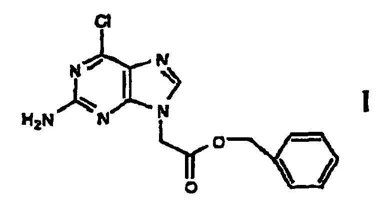 hat.

5. Verfahren nach Anspruch 2, bei dem die Carbamat-geschützte Guanin-Seitenkelteneinhelt aus der Gruppe bestehend aus
(a) einer Verbindung der Formel 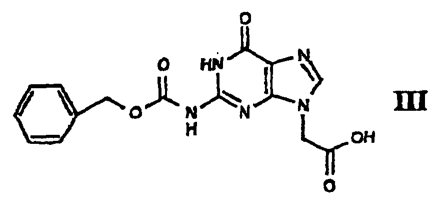
(b) einer Verbindung der Formel 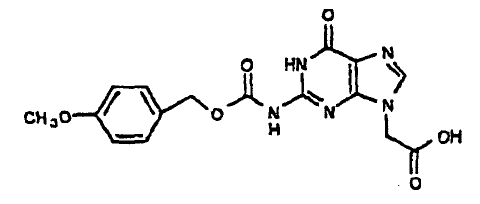 und
(c) einer Verbindung der Formel 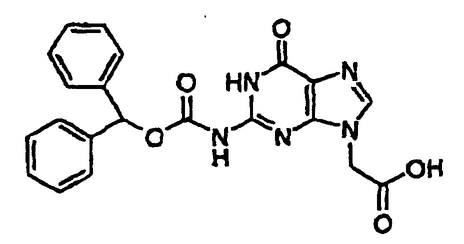 ausgewählt ist.

6. Verfahren nach Anspruch 1, bei dem das Koppeln der geschützten Guanin-Seitenketteneinheit mit einem N-geschützten Ester-Grundgerüst der Aminosäure N-(2-Aminoethyl)glycin die Schritte umfasst:
(a) Bilden eines gemischten Anhydrids der Carbamat-geschützten Guanin-Seitenketteneinheit; und
(b) Umsetzen des gemischten Anhydrids mit dem N-geschützten Ester-Grundgerüst der Aminosäure N-(2-Aminoethyl)glycin, wodurch der Guanin-PNA-Synthonester gebildet wird.

7. Eine vollständig geschützte 2-Amino-6-halopurinverbindung der Formel 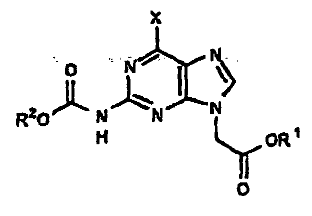
worin X und R¹ die in Anspruch 2 angegebene Bedeutung haben, und
R² die in Anspruch 1 angegebene Bedeutung hat;
und die vollständig geschützte 2-Amino-6-halopurinverbindung der vorstehenden Formel
(i) worin R¹ eine Benzylgruppe und R² die Gruppe CH₃SCH₂CH₂- ist, oder
(ii) worin R¹ eine Benzylgruppe und R² die Gruppe C₆H₅SCH₂CH₂- ist,
und die vollständig geschützte 2-Amino-6-halopurinverbindung der Formel 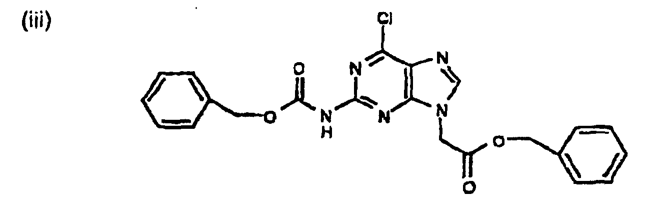 oder

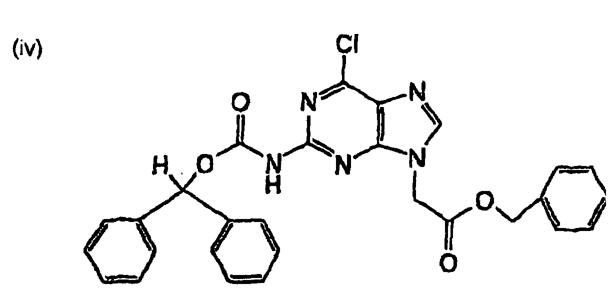

8. Eine Carbamat-geschützte Guanin-Seitenketteneinheit der Formel 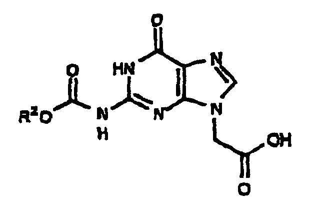
worin R² die in Anspruch 1 angegebene Bedeutung hat,
und die Carbamat-geschützte Guanin-Seitenketteneinheit der Formel 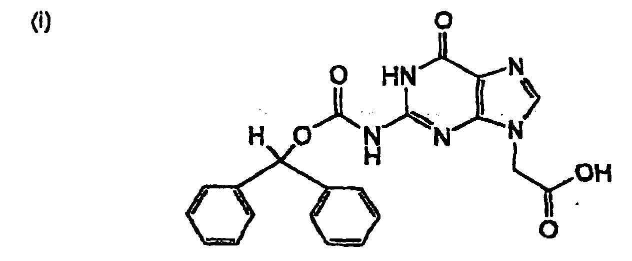 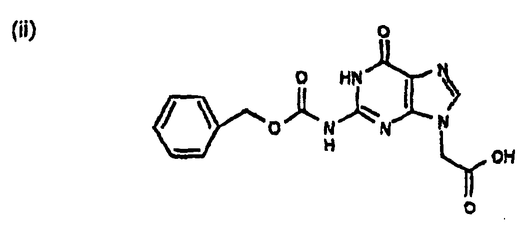 oder

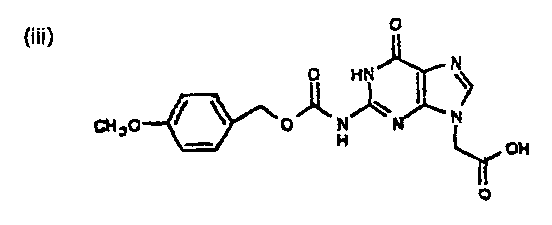

9. Ein Guanin-PNA-Synthon oder dessen Ester der Formel 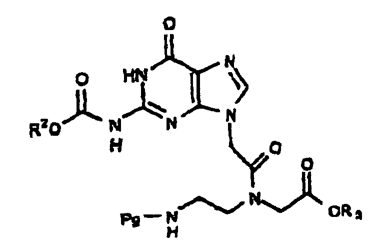
worin Pg und R² die in Anspruch 1 angegebene Bedeutung haben und
R₃ aus der Gruppe bestehend aus einem Wasserstoffatom, einer Methyl- und einer Ethylgruppe ausgewählt íst,
und das Guanin-PNA-Synthon oder dessen Ester der Formel 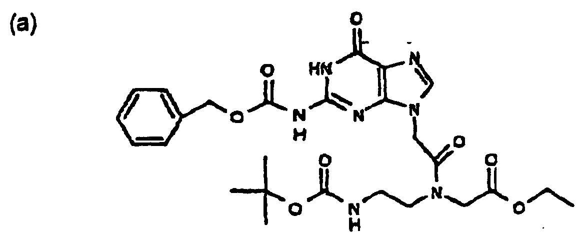 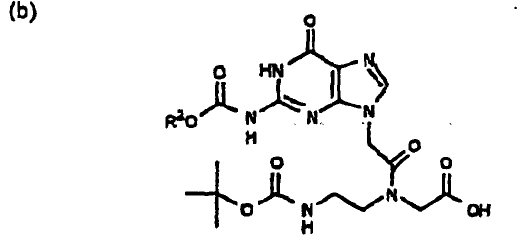
worin R² die in Anspruch 1 angegebene Bedeutung hat: 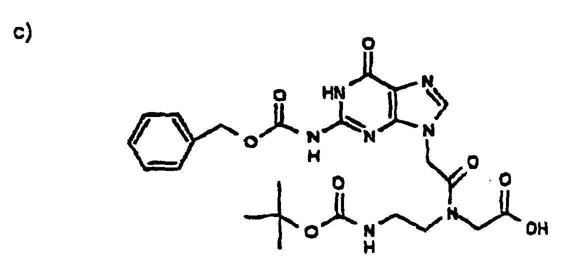 oder

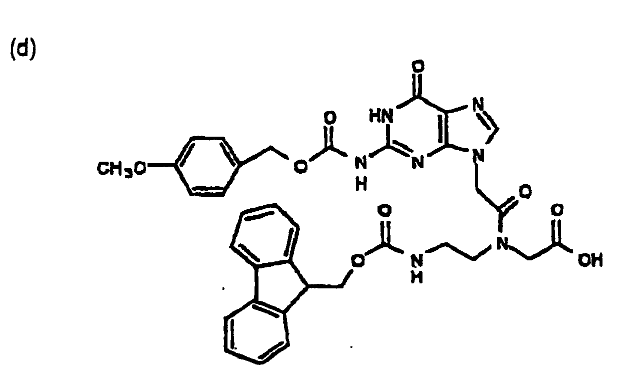

## Revendications

1. Procédé de synthèse d'un synthon guanine de PNA ou de son ester de formule: 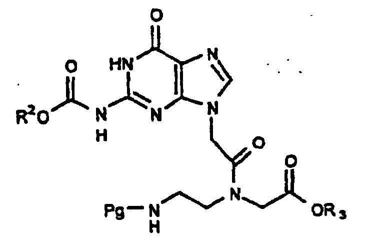 dans laquelle
Pg est un groupe protecteur choisi dans le groupe constitué par les groupes alkyloxycarbonyle, 9-fluorénylméthyloxycarbonyle, 1-méthyl-1-(4-biphényl)éthyloxycarbonyle, 1-méthyl-1-phényléthyloxycarbonyle, triphénylméthyle, 4-méthoxytriphénylméthyle et 4,4'-diméthoxytriphénylméthyle;
R² est choisi dans le groupe constitué par les groupes méthyle, éthyle, 2,2,2-trichloroéthyle, 2-(triméthylsilyl)éthyle, propyle, isopropyle, n-butyle, t-butyle, allyle, 1-isopropylallyle, cinnamyle, 4-nitrocinnamyle, diméthylméthyle,
un groupe benzène ou un de ses dérivés de formule: 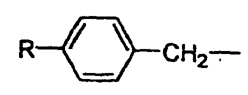 dans laquelle R est un atome d'hydrogène ou un groupe isopropyle, t-butyle ou méthoxy,
et un groupe de formule:
R⁵SCH₂CH₂-
dans laquelle
R⁵ est choisi dans le groupe constitué par les groupes méthyle, éthyle, propyle, isopropyle, n-butyle, t-butyle et phényle, et
R₃ est choisi dans le groupe constitué par un atome d'hydrogène et les groupes méthyle et éthyle;
le procédé comprenant les étapes consistant à:
(i) synthétiser un groupement de chaîne latérale guanine protégé par un carbamate, de formule: 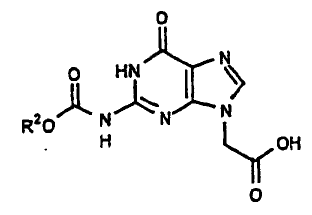 dans laquelle
R² est tel que défini ci-dessus;
(ii) coupler ledit groupement de chaîne latérale guanine protégé par un carbamate à un ester de chaîne principale N-protégé de l'acide aminé N-(2-aminoéthyl)glycine, de formule: 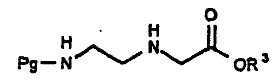 dans laquelle
Pg est tel que défini ci-dessus; et
R³ est choisi dans le groupe constitué par les groupes méthyle et éthyle,
afin de former ledit ester de synthon guanine de PNA; et
(iii) le cas échéant, hydrolyser le groupe ester de chaîne principale dudit ester de synthon guanine de PNA pour former ledit synthon guanine de PNA.

2. Procédé selon la revendication 1 dans lequel le groupement de chaîne latérale guanine protégé par un carbamate de formule: 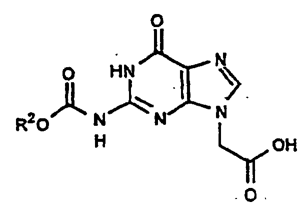 dans laquelle
R² est tel que défini dans la revendication 1,
est préparé par un procédé comprenant les étapes consistant à:
(i) faire réagir le groupe 2-amino exocyclique d'un composé 2-amino-6-halogénopurine partiellement protégé de formule: 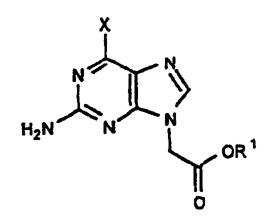 dans laquelle
X est un atome d'halogène, et
R¹ est choisi dans le groupe constitué par les groupes méthyle, éthyle, 2,2,2-trichloroéthyle, 2-(triméthylsilyl)éthyle, 2-(phénylthio)éthyle, propyle, isopropyle, n-butyle, t-butyle, allyle, 1-isopropylallyle, cinnamyle, 4-nitrocinnamyle, et un groupe benzyle ou un de ses dérivés, de formule: 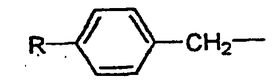
dans laquelle
R est un atome d'hydrogène ou un groupe isopropyle, t-butyle ou méthoxy;
avec au moins un équivalent de phosgène, en présence d'au moins deux équivalents de base non nucléophile, pour produire un composé 2-isocyanato-6-halogénopurine;
(ii) faire réagir ledit composé 2-isocyanato-6-halogénopurine avec un alcool, afin de produire un composé 2-amino-6-halogénopurine totalement protégé de formule: 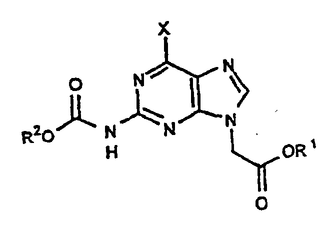 dans laquelle
X et R¹ sont tels que définis ci-dessus, et
R² est tel que défini dans la revendication 1;
(iii) faire réagir ledit composé 2-amino-6-halogénopurine entièrement protégé avec au moins trois (3) équivalents d'un alkylate métallique de 3-hydroxypropionitrile dans un solvant non nucléophile anhydre, afin de produire un sel métallique d'un groupement de chaîne latérale guanine protégé par un carbamate; et
(iv) neutraliser ledit sel métallique dudit groupement de chaîne latérale guanine protégé par un carbamate avec un acide, dans des conditions appropriées pour la protonation à la fois de l'atome d'azote N1 de l'hétérocycle purine et du groupe fonctionnel acide carboxylique de la chaîne principale, afin de former ledit groupement de chaîne latérale guanine protégé par un carbamate.

3. Procédé selon la revendication 2 dans lequel l'alcool est choisi dans le groupe constitué par le méthanol, l'éthanol, le 2,2,2-trichloroéthanol, le 2-(triméthylsilyl)éthanol, le n-propanol, l'isopropanol, le n-butanol, le t-butanol, l'alcool allylique, l'alcool 1-isopropylallylique, l'alcool cinnamylique, l'alcool 4-nitrocinnamylique, le diphénylméthanol,
un alcool benzylique ou un de ses dérivés, de formule: 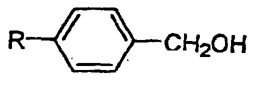 dans laquelle R est un atome d'hydrogène ou un groupe isopropyle, t-butyle ou méthoxy, et
un composé de formule:
R⁵SCH₂CH₂OH
dans laquelle R⁵ est choisi dans le groupe constitué par les groupes méthyle, éthyle, propyle, isopropyle, n-butyle, t-butyle et phényle.

4. Procédé selon la revendication 2 dans lequel la 2-amino-6-halogénopurine partiellement protégée a pour formule:

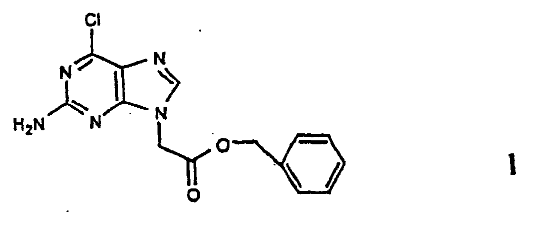

5. Procédé selon la revendication 2 dans lequel le groupement de chaîne latérale guanine protégé par un carbamate est choisi dans le groupe constitué par:
(a) un composé de formule: 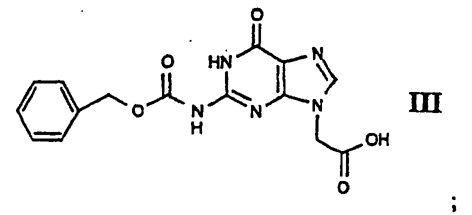
(b) un composé de formule: 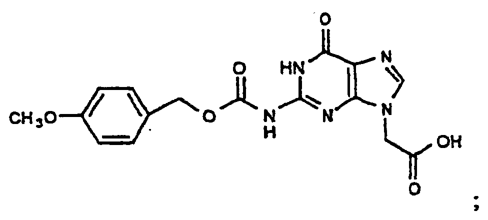 et
(c) un composé de formule:

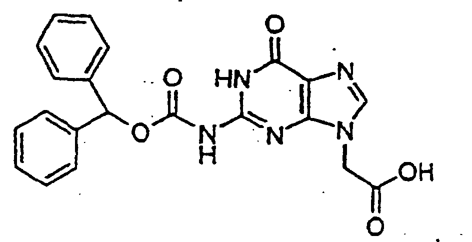

6. Procédé selon la revendication 1 dans lequel le couplage dudit groupement de chaîne latérale guanine protégé à un ester de chaîne principale N-protégé de l'acide aminé N-(2-aminoéthyl)glycine comprend les étapes consistant à:
(a) former un anhydride mixte dudit groupement de chaîne latérale guanine protégé par un carbamate; et
(b) faire réagir ledit anhydride mixte avec ledit ester de chaîne principale N-protégé de l'acide aminé N-(2-aminoéthyl)glycine, afin de former ledit ester de synthon guanine de PNA.

7. Composé 2-amino-6-halogénopurine entièrement protégé de formule: 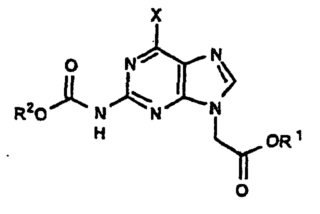 dans laquelle
X et R¹ sont tels que définis dans la revendication 2, et
R² est tel que défini dans la revendication 1;
et ledit composé 2-amino-6-halogénopurine entièrement protégé répondant à la formule ci-dessus:
(i) dans laquelle R¹ est un groupe benzyle et R² est CH₃SCH₂CH₂- ou
(ii) dans laquelle R¹ est un groupe benzyle et R² est C₆H₅SCH₂CH₂-;
et ledit composé 2-amino-6-halogénopurine entièrement protégé répondant à la formule: 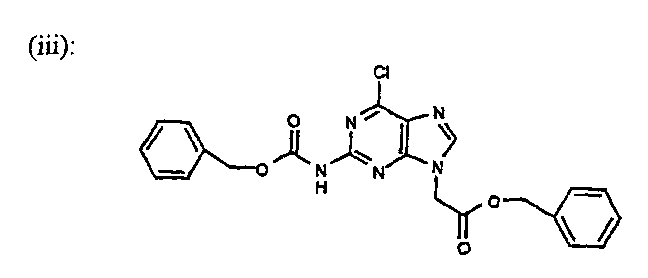 ; ou

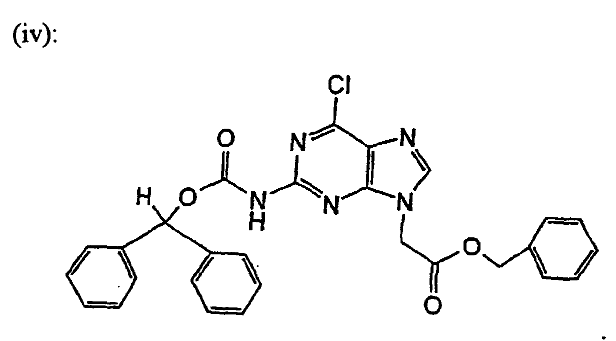

8. Groupement de chaîne latérale guanine protégé par un carbamate, de formule: 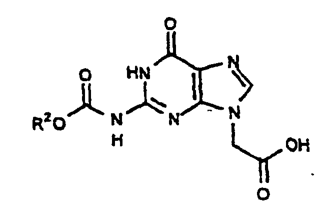 dans laquelle
R² est tel que défini dans la revendication 1;
et ledit groupement de chaîne latérale guanine protégé par un carbamate répondant à la formule: 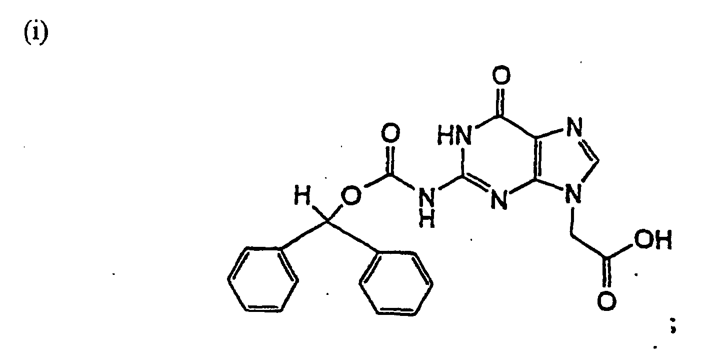 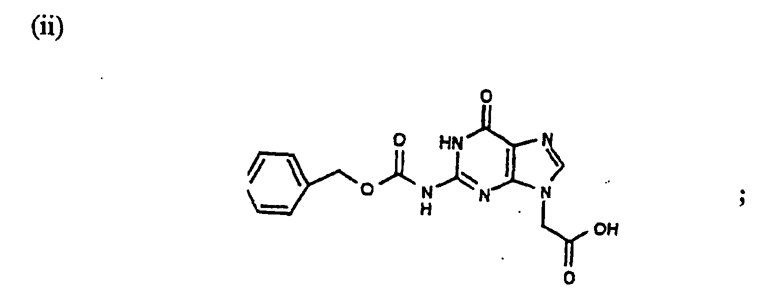 ou

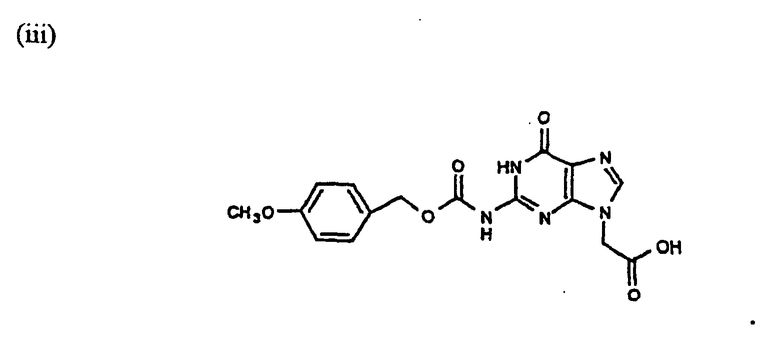

9. Synthon guanine de PNA ou son ester de formule: 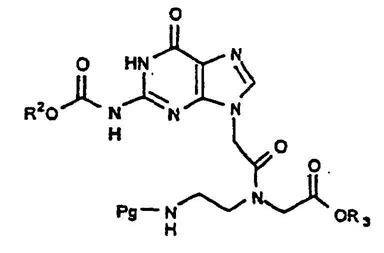 dans laquelle
Pg et R² sont tels que définis dans la revendication 1; et
R₃ est choisi dans le groupe constitué par un atome d'hydrogène et les groupes méthyle et éthyle;
et ledit synthon guanine de PNA ou son ester répondant à la formule: 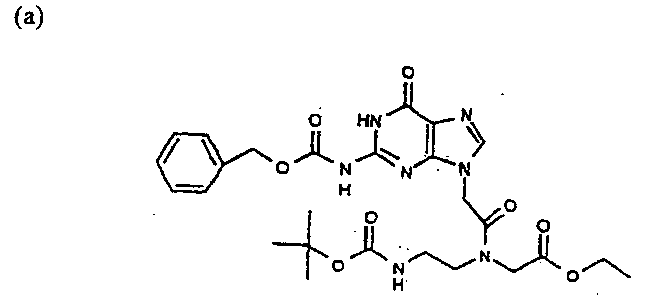 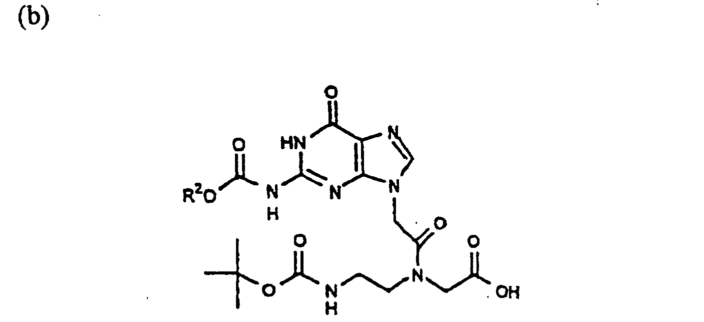 dans laquelle
R² est tel que défini dans la revendication 1; 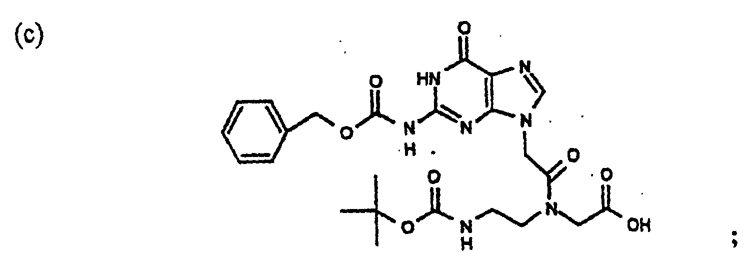
ou
